# EUROPEAN PATENT SPECIFICATION

(11) **EP 1 187 807 B1**
(45) Date of publication and mention of the grant of the patent: **25.07.2007**
(21) Application number: 00925490.5
(22) Date of filing: 26.04.2000
(51) Int. Cl.: C07C 237/22, A61K 47/48, A61K 9/127, C12N 15/88

(54) **BIPOLAR LIPIDS AND THEIR USE FOR THE DELIVERY OF BIOACTIVE SUBSTANCES**
BIPOLARE LIPIDE UND IHRE VERWENDUNG ZUM TRANSPORT BIOAKTIVER SUBSTANZEN
LIPIDES BIPOLAIRES ET UTILISATION DE CES DERNIERS POUR LA LIBERATION DE SUBSTANCES BIOACTIVES

(30) Priority: 26.04.1999 GB 9909582; 13.10.1999 GB 9924257
(43) Date of publication of application: 20.03.2002
(73) Proprietor: Celltech R&D Limited, Slough, Berkshire SL1 4EN (GB)
(72) Inventor: BLAGBROUGH, Ian, Claverton Down, Bath, BA2 7AY (GB); GEALL, Andrew John, Claverton Down, Bath BA2 7AY (GB); EATON, Michael Anthony William, Aston Rowant, Oxfordshire OX9 5SH (GB); NORMAN, Timothy John, Great Missenden, Buckinghamsh. HP16 9AU (GB); BAKER, Terence Seward, The Mayes, Staines, Middlesex TW19 5JF (GB); WEIR, Andrew Neil Charles, Twyford, Berkshire RG10 9DD (GB); CATTERALL, Catherine Fiona, Gerrards Cross, Buckinghamshire SL9 7LH (GB)
(74) Representative: Marsden, John Christopher
(86) International application number: PCT/GB2000/001613
(87) International publication number: WO 2000/064858

(56) References cited:
- WO-A-96/17823
- WO-A-99/08997
- WO-A-99/52858
- FUHRHOP J -H ET AL: "BOLAAMPHIPHILES WITH MANNOSE- AND TETRAALKYLAMMONIUM HEAD GROUPS ASCOATINGS FOR NUCLEIC ACIDS AND POSSIBLE REAGENTS FOR TRANSFECTIONS" CHEMISTRY AND PHYSICS OF LIPIDS,IR,LIMERICK, vol. 43, 1 April 1987 (1987-04-01), pages 193-213, XP000562618 ISSN: 0009-3084

## Description

This invention relates to a series of bipolar lipids and to their use to deliver bioactive substances to cells.

To be effective, many pharmaceutical agents need to be efficiently delivered to the cytoplasm of a eucaryotic cell. For many low molecular weight compounds of low to moderate polarity this is not a problem since such molecules can pass directly through the plasma membrane of the cell and into the cytoplasm. Direct passage is not available to other compounds of greater polarity or high molecular weight and these generally enter the cell by receptor mediated endocytosis or phagocytosis. These mechanisms are not efficient however with all sizes and types of molecule. In particular, large, polyanionic compounds are not readily taken up by cells when delivered to them in aqueous solution.

One general solution to this problem is to couple any poorly transported pharmaceutical agent to a carrier which itself is readily taken up into the cytoplasm of a cell. This is not always satisfactory however, since coupling to the carrier may have an undesirable effect on the metabolism and/or antigenicity of the pharmaceutical agent and/or it may be difficult to recover the desired biological activity from the resulting conjugate once inside the cell.

An alternative solution is to formulate the pharmaceutical agent with a delivery vehicle which is soluble in aqueous solutions but which can also mimic naturally occurring cell membrane constituents. This encourages fusion of the vehicle with a cell membrane and subsequent delivery of any associated pharmaceutical agent to the cytoplasm.

Amphiphilic lipids have frequently been used for this purpose. These typically have a hydrophobic backbone composed of one or more hydrocarbons and a hydrophilic polar head group containing one or more ionisable groups, to facilitate the transport of macromolecules to and across the plasma membrane of cells and into the cytoplasm. The polarity of the head group may be controlled by the selection of the number and/or type of ionisable groups to achieve a range of negatively charged (anionic), neutral or positive charged (cationic) lipids.

For the delivery of polyanions it is generally advantageous to use cationic lipids. The advent of gene therapy and the need to deliver anionic molecules such as nucleic acids to mammalian cells has provided much impetus to the development of this class of lipids. First generation compounds include those with a monocation head group such as N-[1 (2,3-dioleyloxy)propyl]-N,N,N-trimethylammonium chloride [DOTMA; Felgner, P L and Ringold, G M, Nature, 337 387-388 (1989)], 1,2-dimyristyloxypropyl-3-dimethylhydroxyethylammonium bromide, [DMRIE; Zabner, J et al J. Biol. Chem, 270,18997-19007 (1995)], and bola amphiphiles [Fuhrhop J.-H et al. , Chemistry and Physics of Lipids 43,193-213 (1987)] 3β[N-(N¹,N¹-dimethylaminoethane)carbamoyl]cholesterol [DC-Chol; Farhood, H et al, Biochim. Biophys. Acta. 1111, 239-246 (1992)] and those with a polycation head group such as dioctadecyl-amidoglycylspermine [DOGS; Behr, J-P, et al, Proc. Natl. Acad. Sci. 86, 6982-6986 (1989)].

In an effort to improve the properties of these early compounds for in vivo delivery of polyanions many more cationic lipids have been developed in which the nature and size of the hydrophobic backbone and/or the cationic head group have been varied (see for example International Patent Specifications Nos. WO95/21931, WO96/10038, WO96/17823, WO96/18273, WO96/25508, WO96/26179, WO96/41606, WO97/18185, WO97/25339, WO97/3010, WO97/31934 and WO99/52858).

The goal in the development of cationic lipids for in vivo use is to provide a molecule which is simple to use in a clinical setting; which is robust; which forms small stable complexes over wide pH and ionic strength ranges; which is non-toxic; and which is capable of delivering a high concentration of polyanion to a cell.

We have now developed a class of lipid which meets these requirements. Importantly, our lipids are capable of self-assembly and will form stable complexes in aqueous solutions. The lipids are able to efficiently compact polyanions to give defined particle sizes of less than 500nm. The lipid-polyanion complex remains associated over wide pH and ionic strength ranges and is able to efficiently deliver high concentrations of polyanions to cells.

Thus according to one aspect of the invention we provide a bipolar lipid comprising a cationic head (1), a hydrophobic backbone (2) and a hydrophilic tail (3) in which:
(A) the cationic head comprises two or more cationic centres, each centre containing at least one heteroatom which is capable of retaining a positive charge at a pH in the range 2-10 and which is covalently linked to the one or more other centres by one or more carbon-containing spacer groups;
(B) the hydrophobic backbone comprises at least one hydrocarbon chain; and
(C) the hydrophilic tail comprises at least one tail unit selected from synthetic polyols, naturally occurring polyols, poly(alkylene oxides) and derivatives thereof;
each of said components (1) to (3) being covalently linked head (1) to backbone (2) to tail (3) and arranged such that said at least one hydrocarbon chain of the hydrophobic backbone (2) is covalently attached to one of said positive charge retaining heteroatoms in the cationic head (1) and to said at least one tail unit in the hydrophilic tail (3) such that the chain contains at least ten chain-linked atoms between its points of attachment to said positive charge retaining heteroatom and to said at least one tail unit.

In practice, whether a heteroatom will retain a positive charge in the pH range 2-10 as specified above will depend on the nature and number of any other atoms or groups attached to it. Thus particular examples of suitable cationic centres include primary, secondary, tertiary and quaternary amino groups, sulphonium and phosphonium groups.

The number of cationic centres may be varied as desired depending on the intended use of the lipid of the invention. At least two centres will be present, but three, four, five, six, seven, eight or more may be optionally incorporated. More than one type of centre may be present, for example mixtures of amino groups may be accommodated, and/or sulphonium and/or phosphonium groups.

In one general preference each cationic centre is an amino group. Particularly useful amino groups include primary and secondary amino groups. The number of cationic centres in the cationic head (1) will preferably be from three to six.

Each cationic centre will in general be separated from any other centre by spacer groups arranged to link the centres in a linear (straight and/or branched) or cyclic fashion. The overall effect may be a cationic head (1) which has a straight and/or branched linear structure, a cyclic structure, or a mixture of straight and/or branched linear and cyclic structures. More than one type of spacer group may be present in a cationic head (1). Where desired a spacer group may form a terminal group on the cationic head (1), acting as a substituent on a cationic centre rather than a group connecting centres together.

Each spacer group will in general be non-ionic and contain at least one carbon atom. Suitable groups include optionally substituted aliphatic, cycloaliphatic, heteroaliphatic, heterocycloaliphatic, aromatic or heteroaromatic groups.

Particular examples of optionally substituted aliphatic spacer groups include optionally substituted C₁₋₁₀aliphatic chains such as optionally substituted straight or branched C₁₋₆alkylene, C₂₋₆alkenylene or C₂₋₆alkynylene chains.

Heteroaliphatic spacer groups include the aliphatic chains just described but with each chain additionally containing one, two, three or four heteroatoms or heteroatom-containing groups. Particular heteroatoms or groups include atoms or groups L² where L² is as defined below for L¹ when L¹ is a linker atom or group. Each L² atom or group may interrupt the aliphatic chain, or may be positioned at its terminal carbon atom to connect the chain to the atom or group R¹.

Particular examples of aliphatic spacer groups include optionally substituted -CH₂-, -CH₂CH₂-, -CH(CH₃)-, -C(CH₃)₂-, -(CH₂)₂CH₂-, -CH(CH₃)CH₂-, -(CH₂)₃CH₂-, -CH(CH₃)CH₂CH₂-, -CH₂CH(CH₃)CH₂-, -C(CH₃)₂CH₂-, -(CH₂)₄CH₂-, -(CH₂)₅CH₂-, -CHCH-, -CHCHCH₂-, -CH₂CHCH-, -CHCHCH₂CH₂-, -CH₂CHCHCH₂-, -(CH₂)₂CHCH-, -CC-, -CCCH₂-, -CH₂CC-, -CCCH₂CH₂-, -CH₂CCCH₂-, or -(CH₂)₂CC- chains. Where appropriate each of said chains may be optionally interrupted by one or two atoms and/or groups L² to form an optionally substituted heteroaliphatic spacer group. Particular examples include optionally substituted -L²CH₂-, -CH₂L²CH₂-, -L² (CH₂)₂-, -CH₂L²(CH₂)₂-, -(CH₂)₂L²CH₂-, -L²(CH₂)₃- and -(CH₂)₂L²(CH₂)₂- chains. The optional substituents which may be present on aliphatic or heteroaliphatic spacer groups include one, two, three or more substituents selected from halogen atoms, e.g. fluorine, chlorine, bromine or iodine atoms, or hydroxyl, C₁₋₆alkoxy, e.g. methoxy or ethoxy, haloC₁₋₆alkoxy, e.g. halomethoxy or haloethoxy such as difluoromethoxy or trifluoromethoxy, thiol, or C₁₋₆alkylthio e.g. methylthio or ethylthio. Particular examples of substituted spacer groups include those specific chains just described substituted by one, two, or three halogen atoms such as fluorine atoms, for example chains of the type -CH(CF₃)-, -C(CF₃)₂- -CH₂CH(CF₃)-, -CH₂C(CF₃)₂-, -CH(CF₃)- and -C(CF₃)₂CH₂-.

Optionally substituted cycloaliphatic spacer groups in the cationic head (1) include optionally substituted C₃₋₁₀ cycloaliphatic groups. Particular examples include optionally substituted C₃₋₁₀cycloalkylene, e.g. C₃₋₇cycloalkylene, C₃₋₁₀cycloalkenylene e.g. C₃₋₇cycloalkenylene or C₃₋₁₀cycloalkynylene e.g. C₃₋₇cycloalkynylene groups.

Particular examples of cycloaliphatic spacer groups include optionally substituted cyclopropylene, cyclobutylene, cyclopentylene, cyclohexylene, cycloheptylene, 2-cyclobuten-1-ylene, 2-cyclopenten-1-ylene and 3-cyclopenten-1-ylene groups.

Optionally substituted heterocycloaliphatic spacer groups include the optionally substituted cycloaliphatic groups just described but with each group additionally containing one, two, three or four heteroatoms or heteroatom-containing groups L² as just defined.

The optional substituents which may be present on the cycloaliphatic or heterocycloaliphatic spacer groups include one, two, three or more substituents selected from halogen atoms C₁₋₆alkyl, e.g. methyl or ethyl, haloC₁₋₆alkyl, e.g. halomethyl or haloethyl such as difluoromethyl or trifluoromethyl, hydroxyl, C₁₋₆alkoxy, e.g. methoxy or ethoxy, haloC₁₋₆alkoxy, e.g. halomethoxy or haloethoxy such as difluoromethoxy or trifluoromethoxy, thiol, or C₁₋₆alkylthio e.g. methylthio or ethylthio groups.

Optionally substituted aromatic spacer groups include for example monocyclic C₆₋₁₂ aromatic groups, such as optionally substituted phenylene.

Optionally substituted heteroaromatic spacer groups, include for example optionally substituted monocyclic C₁₋₉ heteroaromatic groups containing for example one, two, three or four heteroatoms selected from oxygen, sulphur or nitrogen atoms. Monocyclic heteroaromatic groups include for example five- or six-membered heteroaromatic groups containing one, two, three or four heteroatoms selected from oxygen, sulphur or nitrogen atoms.

Optional substituents which may be present on the aromatic or heteroaromatic spacer groups include one, two, three or more substituents selected from those just described in relation to cycloaliphatic and heterocycloaliphatic spacer groups.

In one general preference each spacer group in the cationic head (1) is preferably an optionally substituted straight or branched C₁₋₆alkylene chain. Particularly useful chains include -(CH₂)₂-, -(CH₂)₃- and -(CH₂)₄-chains.

The hydrophobic backbone (2) in the lipids according to the invention may comprise one or more hydrocarbon chains. Each hydrocarbon may be for example an optionally substituted straight or branched aliphatic or heteroaliphatic chain containing a minimum of ten up to a maximum of around one hundred chain-linked atoms as described in more detail below. The hydrocarbon may be attached either directly or indirectly through a linker atom or group to the cationic head (1). Particular examples of suitable linker groups are those represented by the group L¹ described below.

At least one positive charge retaining heteroatom (as hereinbefire defined) in a cationic centre in the lipids of the invenion is covalently linked to a hydrocarbon chain of the hydrophobic backbone (2). Where desired any other available positive charge retaining heteroatom in the same or any other cationic centre may be additionally linked to the same or other hydrocarbon chains making up the backbone (2). It is generally preferred however to link the backbone (2) and cationic head (1) at one positive charge retaining heteroatom in one cationic centre. Thus a preferred class of lipids according to the invention has one or two hydrocarbon chains as just described indirectly linked through a linker atom or group to a positive charge retaining heteroatom in a cationic centre in the cationic head (1).

Each tail unit in the hydrophilic tail (3) may be attached directly or indirectly through a linker atom or group to a hydrocarbon chain of the hydrocarbon backbone (2). The attachment point may be anywhere on the hydrocarbon chain provided that it is at least ten chain-linked atoms (i.e. at least ten atoms along the chain, excluding branches) from the terminal carbon atom connecting the hydrocarbon chain of the hydrophobic backbone (2) to the cationic head (1). In one general preference the attachment point may be at a terminal carbon atom of a hydrocarbon chain distal to the chain carbon atom attached to the cationic head (1).

A particularly useful group of lipids according to the invention may be represented by the formula (1):

[R¹]m-(L¹)n-R² (1)

wherein R¹ is a hydrocarbon chain optionally substituted by one or more tail units selected from synthetic polyols, naturally occurring polyols, poly(alkylene oxides) and derivatives thereof, provided that at least one hydrocarbon chain R¹ is substituted by at least one such tail unit and that each such tail unit is attached to the hydrocarbon chain to achieve at least a ten chain-linked atom spacing along the chain between the tail unit and the group -(L¹)ₙ-R²;
m is an integer from 1 to 6;
L¹ is a linker atom or group;
n is zero or the integer 1;
R² is an optionally substituted aliphatic, cycloaliphatic, heteroaliphatic, heterocycloaliphatic, aromatic or heteroaromatic group containing two or more cationic centres, such that each [R¹]ₘ-(L¹)ₙ- group is attached to a positive charge retaining heteroatom in a cationic centre in R²;
and the salts, solvates and hydrates thereof.

In the compounds of formula (1), the optionally substituted aliphatic, cycloaliphatic, heteroaliphatic, heterocycloaliphatic, aromatic or heteroaromatic group represented by R² may be an optionally substituted C₁₋₃₀ aliphatic, C₃₋₁₀ cycloaliphatic, C₁₋₃₀ heteroaliphatic, C₃₋₁₀ heterocycloaliphatic, C₈₋₁₂ aromatic or C₁₋₉ heteroaromatic group, containing two or more cationic centres. Particular examples of such groups include those generally and specifically described above in relation to the spacer groups present in the cationic head (1) with the additional presence of one or more cationic centres as defined herein.

In general in the lipids of the invention when the hydrophobic backbone (2) and cationic head (1) are joined indirectly by a linker atom or group, as represented by L¹ in compounds of formula (1) when n is 1, then the linker atom or group may be any multivalent atom or group. Particular examples of suitable linker atoms or groups include those of formula -(Alk¹)ᵣ(X¹)ₛ(Alk²)ₜ- where X¹ is an -O- or -S- atom or a -C(O)-, -C(O)O-, -C(S)-, -S(O)-, -S(O)₂ , -N(R³)- [where R³ is a hydrogen atom, a straight or branched alkyl group such as a methyl or ethyl group or an -Alk¹X¹-chain], -CON(R³)-, -OC(O)N(R³)-, -CSN(R³)-, -N(R³)CO-, N(R³)C(O)O-, -N(R³)CS-, -S(O)N(R³)-, -S(O)₂N(R³)-, -N(R³)S(O)-, -N(R³)S(O)₂-, -N(R³)CON(R³)-, or -N(R³)SO₂N(R³)- group [where any of these groups contains two R³ substituents these may be the same or different]; Alk¹ and Alk² which may be the same or different is each an optionally substituted straight or branched C₁₋₆alkylene, C₂₋₆alkenylene or C₂₋₆alkynylene chain optionally interrupted or terminated by one or more, e.g. one, two or three, carbocyclic or heterocarbocyclic groups and/or heteroatoms or heteroatom containing groups X¹ as just defined, and r, s, and t, which may be the same or different, is each zero or the integer 1, provided that when one of r, s or t is zero at least one of the remainder is the integer 1.

Carbocyclic groups which may interrupt the groups Alk¹ and Alk² include for example optionally substituted C₄₋₈cycloalkyl, e.g. optionally substituted cyclopentyl or cyclohexyl groups, or optionally substituted C₄₋₈cycloalkenyl, e.g. optionally substituted cyclopentenyl or cyclohexenyl groups. Heterocarbocyclic groups include for example carbocyclic groups of the types just mentioned containing one or more heteroatoms or heteroatom-containing groups X¹ as defined above. Optional substituents which may be present on the chains represented by Alk¹ and Alk² and the carbocyclic or heterocarbocyclic groups which can interrupt or terminate them include one, two or three substituents selected from halogen atoms, e.g. fluorine, chlorine, bromine or iodine atoms or C₁₋₃alkyl, e.g. methyl or ethyl, or C₁₋₃alkoxy e.g. methoxy or ethoxy groups.

It will be appreciated that the linker atom or group will be at least divalent in the instance where one hydrocarbon chain in the hydrophobic backbone (2) is attached to it. Where it is desired to attach more than one hydrocarbon chain to the linker the latter will need to be selected with an appropriate valency and this will generally mean that at least one of Alk¹ or Alk² will need to be present in a branched form and with the requisite number of X¹ atoms or groups to achieve the desired coupling.

Particular examples of linker groups include groups of formula -X¹Alk²- where X¹ is as defined above and Alk² is an optionally substituted -CH₂-, -(CH₂)₂-, -(CH₂)₃-, -(CH₂)₄-, -(CH₂)₅- or -(CH₂)₆- chain; groups of formula [X¹]₂Alk¹X¹Alk₂ where Alk¹ is a -CH₂CH < group and X¹ and Alk² are as just defined or a group of formula [X¹]₂Alk¹Alk² where X¹, Alk¹ and Alk² are as just defined.

Each hydrocarbon chain in the hydrophobic backbone (2) of the lipids according to the invention and as represented by R¹ in compounds of formula (1) may be a C₁₀ up to about a C₆₀ hydrocarbon chain, for example a C₁₆ to C₆₀ hydrocarbon chain such as a C₁₈ to C₄₈ hydrocarbon chain.

In particular, the chain may be an optionally substituted C₁₀₋₆₀ aliphatic chain such as an optionally substituted straight or branched C₁₀₋₆₀ alkylene, C₁₀₋₆₀ alkenylene or C₁₀₋₆₀ alkynylene chain. Optional substituents which may be present on such chains include one or more halogen atoms, e.g. fluorine, chlorine, bromine or iodine atoms, or haloC₁₋₆ alkyl, e.g. -CF₃ groups. Where desired each alkylene, alkenylene or alkynylene chain may be interrupted by one or more oxygen or sulphur atoms or optionally substituted C₅₋₇ cycloalkyl, e.g. cyclopentyl or cyclohexyl, C₅₋₇ cycloalkenyl, e.g. cyclopentenyl or cyclohexenyl, -C(O)-, -C(S)-, -C(O)N(R³)-, -C(S)N(R³)-, -N(R³)C(O)-, -N(R³)C(S)-, -C(O)O-, -C(O)S-, -OC(O)N(R³)-, -S(O)-, -S(O₂)-, -S(O)N(R³)-, -S(O)₂N(R³)-, -N(R³)S(O)-, -N(R³)S(O)₂-, -N(R³)C(O)N(R³)-, -N(R³)C(S)N(R³)-, -N(R³)S(O)N(R³)- or -N(R³)S(O)₂N(R³)- groups. Optional substituents which may be present on cycloalkyl or cycloalkenyl groups of this type include one or more halogen atoms or haloalkyl groups as just described. It will be appreciated that when the hydrocarbon chain in the hydrophobic backbone (2) is an alkenylene or alkynylene chain it may have more than one unsaturated group.

Suitable polyol tail units are naturally occurring polyols such as acyclic sugars and derivatives thereof, and synthetic polyols. Particular sugars include acyclic mono- and oligosaccharides. Sugar derivatives include acyclic glycosides in which a non-ionic aliphatic or heteroaliphatic group (for example of the type described herein) is joined to a sugar by a glycosidic linkage. Acyclic monosaccharides include for example open-chain compounds containing three to eight, e.g. five or six, carbon atoms and at least two hydroxyl substituents. Acyclic oligosaccharides include for example at least two open-chain monosaccharides as just defined linked together by a glycosidic linkage. More than one type of open-chain monosaccharide may be present to yield a homo- or heterooligosaccharide.

Alternatively the tail unit may be a poly(alkylene oxide) or derivative thereof, such as poly(ethylene oxide), poly(propylene oxide) or methoxy poly(ethylene oxide).

Each tail unit may be linked directly or indirectly to a hydrocarbon chain in the hydrophobic backbone (2). For indirect linkage a linker atom or group may be employed, for example an atom or group L³ where L³ is as defined above for the linker atom or group L¹. Where the group L³ is multivalent, for example when it is a branched alkylene chain containing more than one X¹ atom or group, more than one hydrophilic hydrocarbon may be attached to it.

A particularly useful group of compounds according to the invention has the formula (1a):

[R⁷]ₚ-(L³)q-[R⁶]ₘ-(L¹)ₙ-R² (1a)

wherein R², L¹, m and n are as defined for formula (1);
R⁶ is a hydrocarbon chain;
L³ is a linker atom or group;
R⁷ is a tail unit selected from synthetic polyols, naturally occurring polyols, poly(alkylene oxides) and derivatives thereof;
q is zero or an integer from one to six;
p is an integer from one to six;
provided that each R⁷ or L³ group, when present, is attached to a group R⁶ to achieve at least a ten chain-linked atom spacing along R⁶ between R⁷ or
L³ and the group -(L¹)ₙ-R²;
and the salts, solvates and hydrates thereof.

In the compounds of formula (1a) the hydrocarbon chain represented by R⁶ may be a C₁₀ up to about a C₆₀ hydrocarbon chain as generally and more particularly described above in relation to the group R¹. The group L³ may be a linker atom or group as just defined.

The cationic head (1) in the lipids according to the invention will preferably be a group R² as described above in relation to the compounds of formulae (1) and (1 a). In groups of this type, R² is preferably a group -WSp¹[WSp²]_{b}WSp³ or -WSp¹[WSp²]_{b}WH in which Sp¹, Sp² and Sp³, which may be the same or different, is each a spacer group as defined above, W is a cationic centre as defined herein and b is zero or an integer from one to six.

In particular groups of this type, the cationic centre W is preferably a -NH-group. Sp¹, Sp² and Sp³, which may be the same or different, is each preferably an optionally substituted C₁₋₆alkylene chain. b is preferably an integer from two to four.

Particularly useful cationic heads (1) in compounds of the invention include those of formula -NH[Sp¹NHSp²]NH₂, -NH[Sp¹NHSp²NHSp²]NH₂ or -NH[Sp¹NHSp²NHSp²]NHCH₃ where each Sp¹ and Sp² group is the same or different and is an optionally substituted C₁₋₆alkylene chain, particularly wherein Sp¹ is -CH₂- and each Sp² is -(CH₂)₃- or -(CH₂)₄-.

In general in the lipids according to the invention the hydrophobic backbone (2) preferably comprises two or, especially one hydrocarbon chain as defined herein. Thus m in formulae (1) and (1a) is preferably an integer 2 or, especially, an integer 1. Each hydrocarbon chain, for example as represented by R¹ and R⁶ in formulae (1) and (1 a) respectively, is preferably linear and in particular is a linear, optionally substituted C₁₆₋₃₈alkylene chain. Optionally substituted C₁₈₋₂₄alkylene chains are particularly useful.

In general each hydrocarbon chain in the hydrophobic backbone (2) is preferably linked indirectly to the cationic head (1) through a linker atom or group. The linker atom or group may be for example an atom or group L¹ as defined herein and thus in the compounds of formulae (1) and (1 a) for example n is preferably the integer 1.

Preferred linkers include those of formula -X¹Alk²- or -[X¹]₂Alk¹X¹Alk²- where X¹, Alk¹ and Alk² are as defined previously. Particularly useful linkers of these types are those wherein Alk² is a -(CH₂)₄-, -(CH₂)₅- or, especially, -(CH₂)₆- chain. X¹ in these linkers is preferably a -CONH-group. Alk¹ when present is preferably a -CH₂-CH < chain.

In another general preference each hydrocarbon chain in the hydrocarbon backbone (2) has two, or especially one, tail unit attached to it. Each tail unit is preferably attached to the terminal carbon atom of the hydrocarbon chain distal to the chain carbon atom attached to the cationic head (1). Preferably the hydrophilic hydrocarbon and hydrocarbon chain are indirectly linked through a linker atom or group. Thus in one particular preference in compounds of formula (1a) q is the integer 1 and p is the integer 1 or 2.

In compounds of this type and in general the group L³ may preferably be an atom or group -X¹-, -X¹Alk¹X¹- or -[X¹Alk¹]₂X¹Alk²X¹-. Particularly useful L³ groups include -NHCO-, -CONH-, -CONH(CH₂)₂NHCO-, or -[CONH(CH₂)₂-]₂NCO (CH₂)₂CONH- groups.

Particularly useful lipids according to the invention are those described in the Examples hereinafter.

The lipids according to the invention may generally be prepared by coupling appropriately functionalised cationic heads (1), hydrophobic hydrocarbons (2) and hydrophilic tails (3) in a predetermined order. Standard chemical coupling techniques may be employed utilising starting materials containing one or more reactive functional groups such as acids, thioacids, anhydrides, acid halides, esters, imides, aldehydes, ketones, alcohols and amines. Illustrative reactions are described in detail in the Examples hereinafter for the preparation of a number of lipids according to the invention and these may be readily adapted using different starting materials to provide other compounds of the invention.

Thus in one general approach a homo- or heterobifunctional hydrocarbon chain may first be coupled to a hydrophilic tail or cationic head and the resulting product coupled as necessary to the remaining component to provide the lipid of the invention.

The homo- or heterobifunctional hydrocarbon chain may be any hydrocarbon chain described herein containing two different reactive functional groups of the types just described. Particularly useful groups include acids and thioacids and reactive derivatives thereof, and amines. These can be used to participate in acylation or thioacylation reactions to couple the hydrocarbon chain to an amine or acid as appropriate in any suitable hydrophilic tail and/or cationic head.

Acylation or thioacylation may be achieved using standard conditions for reactions of this type. Thus, for example the reaction may be carried out in a solvent, for example an inert organic solvent such as an amide, e.g. a substituted amide such as dimethylformamide, an ether, e.g. a cyclic ether such as tetrahydrofuran, or a halogenated hydrocarbon, such as dichloromethane, at a temperature from around ambient temperature to the reflux temperature, optionally in the presence of a base such as an amine, e.g. triethylamine, or a cyclic amine, such as 1,8-diazabicyclo[5.4.0]undec7-ene, pyridine, dimethylaminopyridine, or N-methylmorpholine.

Where an acid is used the acylation may additionally be performed in the presence of a condensing agent, for example a diimide such as 1-(3-dimethylaminopropyl)-3-ethylcarbodiimide or N,N'-dicyclohexylcarbodiimide, advantageously in the presence of a catalyst such as an N-hydroxy compound e.g. an N-hydroxytriazole such as 1-hydroxybenzotriazole or an N-hydroxyimide such as N-hydroxysuccinimide. Alternatively, the acid may be reacted with a chloroformate, for example ethylchloroformate, prior to reaction with the amine.

In the heterobifunctional hydrocarbon chain one of the reactive functional groups may need to be in a protected form prior to any coupling reaction to avoid its unwanted participation in the reaction. Similarly other functional groups when present in the hydrocarbon chain, or the intermediates used to generate the hydrophilic tail and/or the cationic head may need to be in a protected form before these reagents can be used. Conventional protecting groups may be used in accordance with standard practice [see, for example, Green, T. W. in "Protective Groups in Organic Synthesis", John Wiley & Sons, 1991 and the Examples hereinafter].

Suitable heterobifunctional hydrocarbon chains are either known, readily available materials or may be obtained by synthesis using conventional techniques for example as described in the Examples hereinafter. Thus generally a heterobifunctional hydrocarbon chain of any desired length may be synthesised in one or more reactions using appropriately functionalised shorter chains. Thus in one example a shorter chain aldehyde may be reacted with a shorter chain phosphonium salt to yield a longer chain olefin of the desired length. In this particular example the reaction may be carried out in the presence of a base, for example an organometallic base such as an organolithium compound, a hydride such as sodium or potassium hydride or an alkoxide such as a sodium alkoxide e.g. sodium methoxide. The reaction may be performed in a suitable solvent, for example a polar aprotic solvent such as an alkyl sulphoxide, e.g. dimethylsulphoxide at a low temperature, for example around OC° . The starting aldehyde and phosphonium salt may be obtained from known starting alcohols and halides respectively using conventional procedures. Where desired, the olefin obtained above may be hydrogenated using hydrogen and a catalyst, for example Pearlman's catalyst, to yield the corresponding saturated hydrocarbon chain.

Where it is desired to obtain hydrocarbon chains containing one or more heteroatoms or heteroatom-containing groups these may be synthesised from smaller chains containing functional groups which can be chemically coupled, for example by acylation or thioacylation as generally described above.

Suitable functionalised hydrophilic tails or cationic heads for coupling to the heterobifunctional hydrocarbon chain are either readily available or may be synthesised from known materials by conventional methods for example as described in the Examples hereinafter.

The advantageous properties of the lipids according to the invention may be demonstrated using the small scale tests described hereinafter in the Examples. In these the lipids can be shown efficiently to compact any bioactive substance, and to self-assemble with the substance in aqueous solution to yield stable complexes which remain associated over wide pH and ionic strength ranges and which will efficiently deliver the substance to eucaryotic cells.

The lipids can thus be expected to be of use for the delivery of bioactive substances to cells, particularly eucaryotic cells, *in vitro* and especially *in vivo.* Particular general uses to which the lipids may be put thus include for the delivery of bioactive substances to cells in culture, and in human medicine for the delivery of therapeutic or diagnostic agents, or agents which can generate a host immune response for vaccine or other immuno-modulatory purposes. The lipids are particularly well suited for delivering bioactive polyanions, especially nucleic acids, and are of particular use to modify a host's genotype or its expression.

Thus, in another aspect of the invention we provide a lipid complex characterised in that it comprises a bipolar lipid according to the invention in association with at least one bioactive substance.

In the complexes according to the invention, each bioactive substance may be for example a pharmacologically active agent, a diagnostic agent or any agent able to modify the genotype and/or phenotype of a cell.

Particular examples of such substances include bioactive proteins, peptides, polysaccharides, nucleic acids including synthetic polynucleotides, oligonucleotides and derivatives thereof, lipids, glycolipids, lipoproteins, lipopolysaccharides and viral, bacterial, protozoal, cellular or tissue fractions.

Where desired the complexes according to the invention may contain two or more different bipolar lipids of the invention. Especially useful mixtures include those containing two or more bipolar lipids of the invention which differ from each other in the nature of the'hydrophilic tail present in each. The proportion of each lipid in complexes of this type may be manipulated to obtain complexes with different physio-chemical properties, for example overall surface charge and/or particle size, tailored to meet the intended use of the complex. Thus for example in one advantageous lipid complex containing two or more bipolar lipids, one of the lipids has a hydrophilic tail formed by a poly(alkyene oxide) or a derivative thereof as defined herein, while each of the others has a hydrophilic tail formed by a synthetic or naturally occurring polyol as described previously. The proportion of the first poly(alkylene oxide)-containing lipid may be varied in such complexes so that the mole ratio of first lipid to second and other lipids is from 1:10000 to 1:1, advantageously from around 1:1000 to around 1:20, especially around 1:10. Complexes of these types, particularly where the poly(alkylene oxide) is poly(ethylene oxide), may be obtained which advantageously have zero surface charge and do not aggregate when left in solution and which additionally are able to compact a bioactive substance to give small particles of 150nm and below, particularly 100nm and below, especially around 80-85nm.

The lipids according to the invention are particularly suited for delivering polyanions to cells and preferred lipid complexes of the invention thus include lipid-polyanion complexes in which the polyanion may be any of the above-mentioned bioactive substances possessing a net negative charge. Particular polyanions include nucleic acids, for example single or double stranded, circular or supercoiled DNA or RNA and derivatives thereof. Where desired the DNA may be part of a structure such as a plasmid.

The lipid complexes will in general comprise a lipid according to the invention and a bioactive substance in a weight ratio of around 0.1:1 to around 100:1, for example around 1:1 to around 50:1. The complexes may be formed as liquids, by initially mixing one or more bipolar lipids according to the invention, and a bioactive substance together advantageously in an aqueous solvent using conventional procedures. Where desired the solvent may be removed, for example by lyophilisation, to obtain a solid lipid complex.

Where desired the lipid complexes may be targeted lipid complexes in which the lipid complex is assembled with one or more, particularly one targeting molecules, and the invention extends to such assemblies. The targeting molecule may be for example a member of a complementary binding pair, the other member of the pair being present in a mammalian, e.g. human, or other animal, insect, microbial or plant host either attached to a cell membrane or other cell surface or in soluble form and present intracellularly and/or extracellularly. Thus in general the targeting molecule may be a peptide, including a glycopeptide, a polypeptide, protein, including a glycoprotein or phosphoprotein, a carbohydrate, glycolipid, phospholipid, oligonucleotide, polynucleotide or other organic molecule, e.g. a vitamin, which can specifically bind to a receptor, ligand, antigen or other naturally occurring or synthetic organic molecule.

The binding affinity of the targeting molecule for the other member of the complementary pair will be at least 10⁻⁵M, preferably 10⁻⁷M and greater, e.g. around 10⁻⁸M to around 10⁻¹²M. Preferably the targeting molecule will be selective for the other member of the pair and it will not cross-react although absolute specificity is not essential. Advantageously the interaction of the targeting molecule with its ligand leads to delivery of the lipid complex to the cell interior.

Antibodies and antigen-binding fragments and derivatives thereof such as Fab, Fab' and single chain Fᵥ fragments form one particular class of suitable targeting molecules. Usefully the antibody is a whole antibody, particularly an IgG antibody, or a Fab, Fab' or single chain Fᵥ thereof. Advantageously the antibody, fragment or derivative is an internalising antibody, fragment or derivative.

Other examples of suitable targeting molecules include antibody mimetic molecules produced by combinatorial or other synthetic means; interferons, for example interferons α, β and γ; tumour necrosis factors α and β; interleukins, for example interleukins 1 to 15; chemokines, for example MIP-1α, MIP-1β and RANTES; growth factors, for example PDGF, VEGF, EGF, TGFα, TBFβ, GM-CSF, G-CSF, M-CSF, FGF, IGF, bombesins, thrombopoietin, erythropoietin, oncostatin and endothelin 1; peptide hormones, for example LH, FSH, TRH, TSH, ACTH, CRH, PRH, MRH, MSH, glucagon and prolactin; transferrin; lactoferrin; angiotensin; histamine; insulin; lectins; tissue inhibitor of metalloproteinases, for example TIMP-1, TIMP-2 and TIMP-3; apolipoproteins, for example apolipoprotein E; kinins; and vitamins, for example folic acid and vitamin B12. Fragments and other synthetic analogues of these molecules may be used, where these retain or have the appropriate selective binding action. It will be appreciated that the above list is not exhaustive and may be extended to include other naturally occurring binding molecules, including for example the complementary binding partner, or a binding fragment thereof, of each of those mentioned, for example the PDGF receptor, the VEGF receptor and so on.

Similarly, adhesion molecules and their binding partners or binding fragments thereof may be used in the invention as targeting molecules. Particular examples include VLA-4, VMAC-1, fibronectin, LFA-1, MAC-1, ICAM-1, ICAM-2, Lewis X, GMP-140, ELAM-1, S-Lewis X, fibrinogen, GPIIb/IIIa, CD28, B7, CD40, CD402L, CD4, laminin, VLA-1, VLA-2, VLA-3 and VLA-6.

Other examples of suitable targeting molecules include monosaccharides and oligosaccharides such as galactose, lactose and mannose.

The targeting molecule may be present in the targeted lipid complex either non-covalently associated with or covalently linked to a bipolar lipid of the invention or to any other lipid or other component which may be present as part of a formulation as described below. Covalently linked targeted lipid complexes may be obtained by coupling together a targeting molecule and a bipolar or other lipid or other component using standard chemical coupling techniques and appropriate functional groups present in the starting materials, for example as described above for the preparation of the bipolar lipids of the invention.

The lipid complexes according to the invention including targeted lipid complexes, may be formulated with other materials such as one or more other lipids or other pharmaceutically acceptable carriers, excipients or diluents and the invention extends to such compositions. In this aspect of the invention the "other" lipid may be for example selected from any known neutral and/or cationic lipid, for example selected from those described herein in the introduction to this specification and also especially including DOPE and other cholesterol derivatives such as cholesterol hemisuccinate. Particularly useful formulations of this type are those wherein the bipolar lipid of the invention has a poly(alkylene oxide) tail, especially a poly(ethylene oxide) tail.

Particular compositions include liposome formulations, prepared using conventional liposome technology. Otherwise, the compositions may take any other form suitable for oral, buccal, parenteral, nasal, topical or rectal administration, or a form suitable for administration by inhalation or insufflation.

For oral administration, the compositions may take the form of, for example, tablets, lozenges or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents, emulsifying agents, non-aqueous vehicles and preservatives. The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

The complexes of the invention may be formulated for parenteral administration by injection, including bolus injection or infusion or particle mediated injection. Formulations for injection may be presented in unit dosage form, e.g. in glass ampoule or multi dose containers, e.g. glass vials or a device containing a compressed gas such as helium for particle mediated administration. The compositions for bolus injection or infusion may take such forms as suspensions, solutions or emulsions in oily or aqueous vehicles, and may contain formulatory agents such as suspending, stabilising, preserving and/or dispersing agents. Alternatively, the complex may be in powder form for constitution with a suitable vehicle, e.g. sterile pyrogen-free water, before use. For particle mediated administration the complex may be coated on particles such as microscopic gold particles.

In addition to the formulations described above, the complexes may also be formulated as a depot preparation. Such long acting formulations may be administered by implantation or by intramuscular injection.

For nasal administration or administration by inhalation, the complexes may be conveniently delivered in the form of an aerosol spray presentation for pressurised packs or a nebuliser, with the use of suitable propellant, e.g. dichlorodifluoromethane, trichlorofluoromethane, dichlorotetrafluoroethane, carbon dioxide or other suitable gas or mixture of gases.

The complexes may, if desired, be presented in a pack or dispenser device which may contain one or more unit dosage forms containing the active ingredient. The pack or dispensing device may be accompanied by instructions for administration.

The quantity of lipid complex required for any particular application will to a large extent depend on the nature of the bioactive substance being delivered. Another important factor will include whether the lipid complex is intended for *in vitro* or *in vivo* use. If the latter the route of administration and particular formulation chosen as well as factors such as the age and condition of the subject will govern the quantity of lipid complex used. In general however up to around 50 mg of lipid complex can be used for every kilogram of body weight.

The following Examples illustrate the inventions. In the Examples, the following abbreviations are used: THF - tetrahydrofuran; Bn - benzyl; DMSO- dimethylsulphoxide; DMF - dimethylformamide. LDA - lithium diisopropylamide; Ac - acetyl; MeOH - methanol; DCM - dichloromethane; BOC - t-butyloxycarbonyl;

The compounds of the invention prepared in these Examples are shown below and numbered (14), (19) and (24);
(14)*N*-(aminopropylaminobutylaminopropylaminohexyl)-24-(glucuronylamino) tetracosanamide 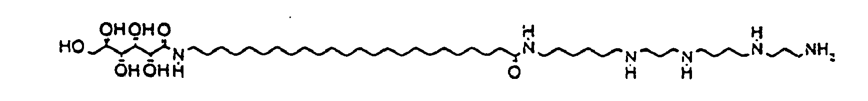
(19) *N*-Aminopropylaminobutylaminopropyl-*N'*-glucuronyl-1,24-diaminotetracosane 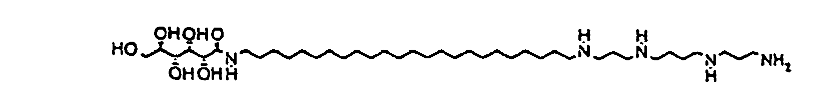
(24) *N*-aminopropylaminobutylaminopropyl-*N*'-glucuronyl-1,36-diaminohexatriacontane

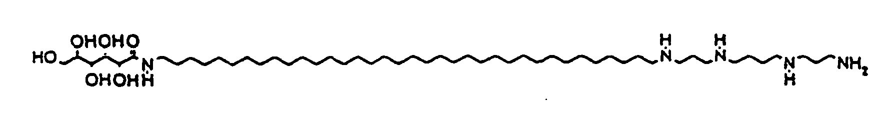

### (1) 12-Aminododecanol hydrochloride

NH₂(CH₂)₁₁ CH₂OH.HCl

12-Aminododecanoic acid (21.52 g, 100 mmol) was suspended in THF (100 cm³) and borane-THF complex (500 mmol, 1M solution) added. The reaction was left overnight and carefully quenched with methanol before removal of the solvent. The residues were suspended in 1M HCl (500 cm³) and heated at 40°C for 1hr and left overnight. The white solid was filtered off, washed with cold 1M HCl, and the product recrystallised from 1M HCl. Yield 18.70 g (79%). Mp 120°C softens, 169°C liquid.

C₁₂H₂₈N₁O₁C1.1/5 H₂O requires C: 59.70%, H: 11.86%, N: 5.80%. Found: C: 59.65%, H: 11.82%, N: 5.76%. C₁₂H₂₇N₁O₁ requires 201. Found ES⁺: MH⁺ 202.1 (100%). δ_{H}(CD₃CO₂D) 3.64 (2H, t, C*H*₂O), 3.06 (2H, t, NC*H*₂), 1.73 (2H, m, C*H*₂CH₂O), 1.57 (2H, m, NCH₂C*H*₂), 1.2-1.5 (16H, m, C*H*₂).

### (2) 12-(Dibenzylamino)dodecanol

Bn₂N(CH₂)₁₁ CH₂OH

To 1 (15 g, 63.2 mmol) suspended in a mixture of dichloromethane (150 cm³) and saturated sodium carbonate in water (150 cm³) was slowly added benzyl bromide (33.7 g, 23.5 cm³, 189.6 mmol). The suspension cleared and reaction was complete after 4hr. Aqueous ammonia (0.880,30 cm³) was added and the reaction left overnight. The organic layer was dried (magnesium sulphate) and evaporated to dryness. The residues were dissolved in refluxing hexane and crystallised at -20°C to yield (18.03 g, 75%).

Mp 45°C. C₂₆H₃₉N₁O₁ requires C: 81.84%, H: 10.30%, N: 3.67%. Found: C: 81.64%, H: 10.24%, N: 3.54%. C₂₆H₃₉N₁0₁ requires 381. Found ES+: MH⁺ 382 (100%). δ_{H} (CDCl₃) 7.1-7.6 (10 H, m, Ar), 3.64 (2H, t, C*H*₂O ), 3.56 (4H, s, ArC*H*₂), 2.41 (2H, t, NC*H*₂), 1.1-1.8 (22H, dm, C*H*₂).

### (3) 12-(Dibenzylamino)dodecanal

Bn₂N(CH₂)₁₁CHO

To a solution of anhydrous dimethylsulphoxide (30 mmol, 2.13 cm³) in dichloromethane (200 cm³) at -78°C was added carefully oxalyl chloride (2.6 cm³, 30 mmol) in dichloromethane (60 cm³). After 15mins 2 (10 g, 26 mmol) was added in dichloromethane (60 cm³) and the reaction stirred for 20 mins at -78°C. Triethylamine (28 cm³) was added dropwise to the cold reaction. A precipitate formed and after 15mins the reaction was allowed to reach room temperature. Water (100 cm³) was added to the reaction that was extracted with dichloromethane. The organic layers were washed with water, dried (magnesium sulphate) and evaporated to dryness. The residue was chromatographed (SiO₂, hexane-10% ethyl acetate in hexane) to give the product as an oil (7.97 g, 80%). This compound is unstable and should be used on the day of preparation.

I.R. 1725 cm⁻¹ (COH). C₂₆H₃₇NO requires 379.29. Found ES+: MH⁺ 380.29. δ_{H}(CDCl₃) 1.32(14H, br, (CH₂)₇(CH₂)₂N), 1.61 (4H, 2xp, *CH₂CH₂N,* CH₂CH₂CO), 2.43, 2.44 (4H, 2xt, CH₂N, CH₂CO), 3.60 (4H, s, CH₂Ph), 7.2-7.5 (10H, m, Ph), 9.78 (1H, t, COH). δ_{C} (CDCl₃) 22.0, 26.9, 27.1, 29.0, 29.3, 29.4, 29.5 (9C, (CH₂)₉CH₂N), 43.8 (1C, CH₂COH), 53.3 (1C, CH₂N), 58.2 (2C, CH₂Ph), 126.6 (2C, CH(CH)₂C), 128.0 (4C. CHC), 128.6 (4C, CHCHC), 140.0 (2C, CCH₂N), 202.3 (1C, COH).

### (4) 11-(Carboxyundecyl)triphenylphosphonium bromide

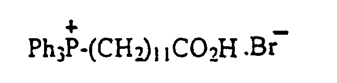

To 12-bromododecanoic acid (3.000 g, 10.7 mmol) suspended in acetonitrile (12 cm³) was slowly added triphenylphosphine (2.818 g, 10.7 mmol). The reaction was heated at 100°C (no condenser) with argon blowing over the flask until the reaction was a fusion, then maintained at 100°C (with condenser) for 24hrs. The warm residues were dissolved in acetonitrile (18 cm³) and added dropwise to rapidly stirred cold (dry ice) diethyl ether. The white precipitate formed was then filtered off and the phosphonium salt dried (5.353 g, 92%).

Mp 110-112°C. C₃₀H₃₈O₂PBr requires C: 66.54%, H: 7.07%. Found: C: 66.42%, H: 7.10%. δ_{P} (CDCl₃) 24.3 (s). δ_{H} (CDCl₃) 1.05-1.30 (12 H, br, (C*H*₂)₆(CH₂)₂CO₂H), 1.53 (6H, br, (C*H*₂)₂CH₂P, C*H*₂CH₂CO₂H), 2.28 (2H, t, CH₂CO₂), 3.55 (2H, br, CH₂P), 7.6-7.8 (15H, m, Ph). δ_{C} (CDCl₃) 22.1, 22.3, 22.8, 24.5, 28.8, 28.9, 30.0, 30.2 (10C, (*C*H₂)₁₀CO₂H), 34.2 (1 C, CH₂P), 117.3, 118.7 (3 C, CP), 130.3, 130.5 (6C, CHCHCP), 133.3, 133.5 (6C, CHCP), 134.9 (3C, *C*H(CH)₂CP), 177.4 (1C, CO₂H).

### (5) 24-(Dibenzylamino)-12-tetracosenoic acid

Bn₂N-(CH₂)₁₁-CH=CH-(CH2)₁₀-CO₂H

4 (13.52 g, 25 mmol) was dissolved in dry DMSO (40 cm³) under argon at -0°C (no DMSO solidification). 2.2 equivalents of 2.OM LDA (25 cm³) were added, the solution turning orange. The reaction was left at 0°C for 30 min, and to the now dark orange solution was added a solution of 3 (7.97 g, 21 mmol) in dry THF (30 cm³). The solution was maintained at 0°C for 4 hours then added to 2M HCl (50 cm³). The aqueous layer was extracted with dichloromethane, the fractions combined, dried (MgSO₄) and the solvent removed to yield the crude material as a pale yellow gum. Silica column chromatography (30-100% ethyl acetate in hexane) yielded the desired product (6.20 g, 53%), as a pale yellow gum.

C₃₈H₅₉NO₂ requires 561.46. Found ES+: MH⁺ 562.53, ES-: (M-H⁺)⁻560.55. δ_{H}(CDCl₃) 1.26 (30H, br, (C*H*₂)₈CH₂CH=CHCH₂(C*H*₂)₇), 1.42-1.72 (4H, m, C*H*₂CH₂CO₂H, CH₂CH₂N), 2.02 (4 H, dxt, C*H*₂CH=CHC*H*₂, 2.34 (2H, t, C*H*₂CO₂H), 2.46 (2H, t, CH₂N), 3.65 (4H, s, CH₂Ph), 5.36 (2H, t, CH=CH), 7.2-7.4 (10H, m, Ph). δ_{C} (CDCl₃) 25.0, 26.4,27.2, 29.3, 29.6 (19C, (CH₂)₁₀CH=CH(CH₂)₉), 34.5 (1C, CH₂CO₂H), 52.9 (1C, CH₂N), 57.7 (2C, CH₂Ph), 127.0 (2C, CH(CH)₂C, 128.2 (4C, CHC), 129.1 (4C, CHCHC), 129.9 (2C, CH=CH), 138.6 (2C, CCH₂N), 179.2 (1C, CO₂H).

### (6) 24-Aminotetracosanoic acid

NH₂(CH₂)₂₃CO₂H

5 (6.2 g) under an atmosphere of hydrogen was heated at 60°C overnight in glacial acetic acid using Pearlman's catalyst (10% w/w). The reaction was filtered through glass fibre and evaporated to dryness. The product was crystallised from acetic acid / ether (4.2 g, 100%). The product was subjected to high vacuum to remove traces of acetic acid.

Mp 151-155°C. C₂₄H₄₉NO₂.0.75 CH₃CO₂H requires C: 71.44%, H: 12.23%, N: 3.27%. Found: C: 71.43%, H: 12.15%, N: 3.26%. C₂₄H₄₉NO₂ requires 383.38. Found ES⁺: MH⁺ 384.29. δ_{H} (CD₃OD + TFA) 1.32 (38H, br, (C*H*₂)₁₉(CH₂)₂ NH₂), 1.65 (4H, br, C*H*₂CH₂NH₂, C*H*₂CH₂CO₂H), 2.33 (2H, t, C*H*₂CO₂H), 2.74 (2H, m, C*H*₂NH₂). δ_{C} (CD₃OD + TFA) partial 33.8 ( 1C, CH₂CO₂H), 35.3 (1C, CH₂NH₂).

### (7) 24-(Glucuronylamino)tetracosanoic acid

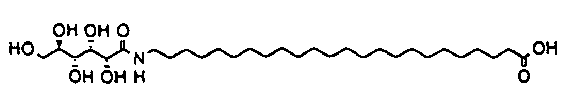

A suspension of 6 (792 mg, 2.064 mmol), δ-gluconolactone (1.839 g, 10.32 mmol) and 1,8-diazabicyclo(5.4.0.)undecene (DBU) (4.2 g, 30.9 mmol) in dry methanol (90 cm³) were heated at 60°C for approximately 10 minutes until all solids had dissolved. The solution was left at room temperature overnight, then the solvent removed. The residues were taken up into water (5 cm³) and acidified to pH 1 with 1M HCl to precipitate out the desired compound. This was filtered off and dried to yield a white solid (765 mg, 66%). Silica tlc R_{f} 0.35, ninhydrin negative (1:1:1 methanol:acetic acid:dichloromethane).

I.R. 1581 cm⁻¹ (CO₂⁻), 1639 cm⁻¹ (CONH). δ_{H} (DMSO) 1.32 (42 H, br, (C*H*₂)₂₁CH₂CO₂H), 2.27 (2 H, t, C*H*₂CO₂H), 3.15 (2 H, m, CH₂N), 3.3-3.8 (4 H, m, C*H*OH), 4.0-4.1 (2 H, m, CH₂O).

### (8) 24-(Peracetylolucuronylamino)tetracosanoic acid

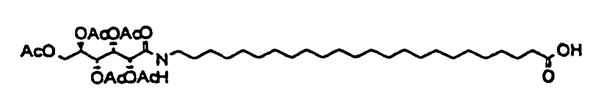

To 7 (765 mg, 1.362 mmol) dissolved in dry pyridine (20 cm³) was added acetic anhydride (20 cm³). The solution was stirred under argon overnight and water (50 cm³) added slowly. The solution was extracted with dichloromethane and the dichloromethane then washed with HCl pH 3 (2 x 20 cm³) and water (5 x 30 cm³). The organics were dried (MgSO₄) and the solvent removed to yield the penta-acetylated product as a white solid (940 mg, 89%). Alumina tlc Rf 0.15 (15% methanol in dichloromethane).

C₄₀H₆₉NO₁₃ requires 771.48. Found ES+: MH⁺ 772.07, MNa⁺ 794.25. ES-: (M-H⁺)⁻ 770.65. δ_{H} (CDCl₃) 1.26 (38 H, br, (CH₂)₁₉(CH₂)₂CO₂H), 1.64 (4 H, m, CH₂CH₂CO₂H, CH₂CH₂NH), 2.07, 2.11, 2.13, 2.21 (15 H, s, CH₃CO), 2.35 (2 H, t, C*H₂*CO₂H), 3.24 (2 H, m, C*H*₂NH), 4.30 (2 H, 2 x dxd, CH₂OAc), 5.05 (1 H, q, C*H*(OAc)CH₂OAc), 5.32 (1 H, d, CH(OAc)CONH), 5.46 (1 H, t, CH(OAc)CH(OAc) CH₂OAc), 5.70 (1 H, t, CH(OAc)CH(OAc)CONH), 6.42 (1 H, t, NH). δ_{C} (CDCl₃) 20.4, 24.5, 26.6, 28.8-29.5 (26 C, (*C*H₂)₂₁CH₂CO₂H, CH₃CO), 33.8 (1 C, *C*H₂CO₂H), 39.3 (1 C, CH₂NH), 61.3 (1 C, CH₂OAc), 68.5, 68.9, 69.1, 71.5 (4 C, CHOAc), 165.8 (1 C, CONH), 160.0, 169.5, 169.7, 170.4 (5 C, CH₃CO), 178.6 (1 C, CO₂H).

### (9) N,N'-bis(t-Butyloxycarbonyl)-N-t-butyloxycarbonylaminopropyl-N'-aminopropyl-1,4-diaminobutane

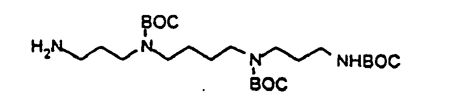

To spermine (3.704 g, 18.31 mmol) dissolved in methanol (200 cm³) at -78°C was added ethyl trifluoroacetate (2.601 g, 18.31 mmol) over 30 minutes. The solution was stirred for a further 30 minutes at -78°C then warmed to 0°C over 1 hour. tert-butyl dicarbonate (15.981 g, 73.23 mmol) was then added as a solid, the reaction allowed to warm to ambient temperature over 1 hour, and a further 2 hours later conc. ammonium hydroxide (approx. 40 cm³) added until a pH > 11 was achieved. The reaction was stirred overnight at ambient temperature, the solvents removed and the residues purified by silica chromatography (70:10:1 DCM:MeOH:NH₄OH) to yield the product 4.240 g, 46% as a pale yellow oil.

C₂₅H₅₀N₄O₆ requires 502.4. Found ES+: MH⁺ 503.4. δ_{H} (CDCl₃) 1.44 (31H, m, C(CH₃)₃, NCH₂(C*H*₂)₂CH₂N), 1.64 (4H, m, NCH₂C*H*₂CH₂N], 3.14 (2H, t, C*H*₂NH₂), 3.0-3.4 (10H, m, CH₂N).

### (10) N-t-Butyloxycarbonyl-N-[t-butyloxycarbonylaminopropyl(t-butyloxycarbonyl)aminobutyl(t-butyloxycarbonyl)aminopropyl-N',N'-dibenzyl-1,6-diaminohexane

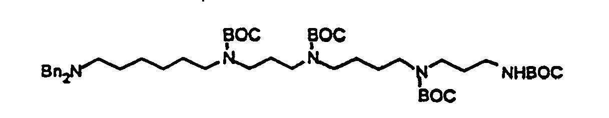

9 (535 mg, 1.064 mmol), 6-(dibenzylamino) hexanal (286 mg, 0.968 mmol) (synthesised as for 3 from 6-aminohexanol) and Na₂SO₄ were stirred overnight in anhydrous methanol (50 cm³) at ambient temperature under argon. To the filtered reaction was added sodium triacetoxy borohydride (410 mg, 1.94 mmol) and the reaction left for 3 hours at which point water (0.5 cm³) was added and the reaction left overnight to quench excess reducing agent. To the reaction were added tert-butyl dicarbonate (633 mg, 2.903 mmol) and triethylamine (490 mg, 4.838 mmol) and the reaction left for 3 hours. The solvent was removed and the residues purified by silica column chromatography eluting with 25-50% ethyl acetate in hexane to yield the desired compound 311 mg, 36% as a colourless gum.

C₅₀H₈₂N₅O₈ requires 881.6. Found ES+: MH⁺ 882.5. δ_{H} (CDCl₃) 1.1-1.8 (52H, m, (CH₃)₃C, Bn₂NCH2(C*H*₂)₄, C*H*₂CH₂N), 2.39 (2H, t, Bn₂NC*H*₂), 3.0-3.4 (14H, br, CH₂N), 3.54 (4H, s, CH₂Ph), 7.2-7.4 (10H, m, Ph).

### (11) N-t-Butyloxycarbonyl-N-[t-butyloxycarbonylaminopropyl(t-butyloxycarbonyl)aminobutyl(t-butyloxycarbonyl)aminopropyl-1,6-diaminohexane

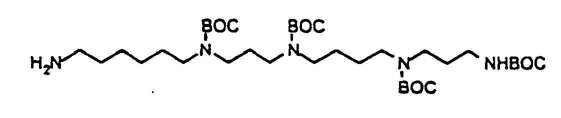

To 10 (311 mg) in tert-butanol at 40°C was added Pearlman's catalyst (100 mg) and the atmosphere changed to hydrogen. Hydrogenation was left for two days at 40°C, the catalyst filtered off and the solvent removed. The residues were purified by silica chromatography eluting initially with 100:10:0 DCM:MeOH:NH₄OH to bring off the impurities then with 100:10:1 to bring off the desired primary amine as a colourless clear oil, 163 mg, 66%.

C₃₆H₇₁N₅O₈ requires 701.5. Found ES+: MH⁺ 702.4. δ_{H} (CDCl₃) 1.2-1.8 (52H, m, (CH₃)₃C, H₂NCH₂(CH₂)₄, C*H*₂CH₂N), 2.88 (2H, br, C*H*₂NH₂), 3.0-3.4 (14H, m, CH₂N).

### (12) N-[t-butyloxycarbonylaminopropyl(t-butyloxycarbonyl)aminobutyl(t-butyloxycarbonyl)aminopropyl(t-butyloxycarbonyl)aminohexyl]-24-(peracetylglucuronylamino)tetracosanamide

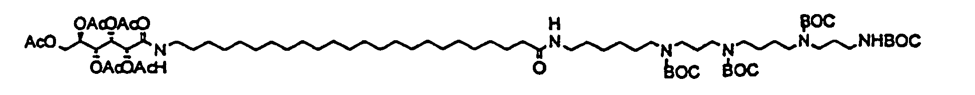

8 (96 mg, 0.125 mmol) in anhydrous dichloromethane (10 cm³) were added 1-(3-dimethylaminopropyl)-3-ethylcarbodiimide hydrochloride (48 mg, 0.250 mmol), N-hydroxysuccinimide (22 mg, 0.187 mmol) and the reaction left overnight. To this were then added 11 (92 mg, 0.131 mmol) and triethylamine (63 mg, 0.624 mmol) and the reaction again left overnight. The solvents were removed and the residues purified by silica gradient chromatography eluting with 50-70% ethyl acetate in hexane to yield the product, 157 mg, 86% as a white solid.

δ_{H} (CDCl₃) 1.24 (44H, br, (C*H*₂)₂₀CH₂CO, N(CH₂)₂(C*H*₂)₂(CH₂)₂N), 1.44 (50H, br+m, (CH₃)₃C, C*H*₂CH₂N), 2.04-2.20 (15H, 5xs, CH₃CO), 2.29 (2H, t, CH₂CO), 3.0-3.4 (18H, m, NCH₂), 4.12, 4.31 (2H, m, CH₂OAc), 5.05 (1H, m.

C*H*CH₂OAc), 5.29 (1H, d, NHCOC*H*OAc), 5.44, 5.67 (2H, 2xt, AcOCH₂CHOAc(C*H*OAc)₂), 6.06 (2H, br, NHCO).

### (13) N-[t-butyloxycarbonylaminopropyl(t-butyloxycarbonyl)aminobutyl(t-butyloxycarbonyl)aminopropyl(t-butyloxvcarbonyl)aminohexyl]-24-(glucuronylamino)tetracosanamide

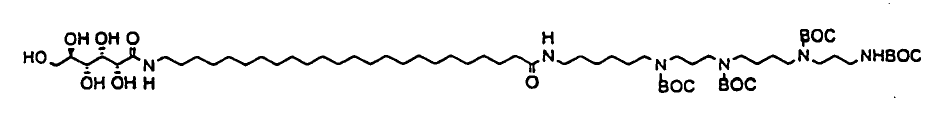

To 12 (156 mg) dissolved in methanol (10 cm³) was slowly added with stirring NH₄OH (4 cm³) until the solution started to become cloudy. After approximately 20 minutes an additional 3 cm³ of methanol was added to dissolve some of the forming white precipitate. The solution / suspension was stirred for a total of one hour at which point all solvents were removed. The residues were purified by reverse phase chromatography eluting with 2:6:1 CH₂Cl₂:MeOH:NH₄OH to yield a colourless solid, 123 mg, 92%.

C₆₆H₁₂₈N₆O₁₅ requires 1244.9 Found ES⁺: MNa⁺ , 1267.8. δ_{H} (CD₃OD) 1.28 (44H, br, NH(CH₂)₂(C*H*₂)₂₀, N(CH)₂(C*H*₂)₂(CH₂)₂N), 1.4-1.9 (50H, brm, (CH₃)₃C, C*H*₂CH₂N), 2.16 (2H, t, CH₂CO), 3.0-3.4 (18H, m, CH₂N), 3.6-3.9 (4H, m, C*H*OH), 4.09,4.19 (2H, 2xm, C*H*₂OH).

### (14) N-(aminopropylaminobutylaminopropylaminohexyl)-24-(glucuronylamino)tetracosanamide

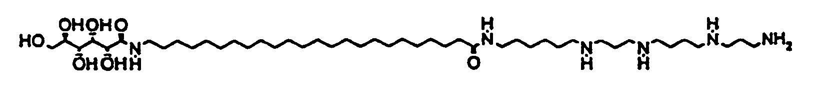

13 (120 mg) was treated with 96:4 trifluoroacetic acid : water for 20 minutes and the solvents removed to yield a solid which was taken up into water, filtered through a 0.2µm polypropylene filter and freeze dried to give the product in quantitative yield as a white solid.

C₄₆H₉₆N₆O₇ requires 844.7 Found ES⁺: MH₂²⁺ , 423.5. δ_{H} (CD₃OD) 1.28 (44H, m, (CH₂)₂₀CH₂CO, N(CH₂)₂(*CH*₂)₂(CH₂)₂N), 1.5-1.8 (6H, m, C*H*₂CH₂N), 1.81 (4H, m, NCH₂(C*H*₂)₂CH₂N), 2.10 (2H, m, CH₂CO), 2.16 (4H, m, NCH₂C*H*₂CH₂N), 2.95-3.30 (18H, m, NCH₂), 3.55-3.80 (4H, m, C*H*OH), 4.08, 4.20 (2H, 2xm, CH₂OH).

### (15) 23-Azido-1-(1,3-dioxalan-2-yl)tricosa-11-ene

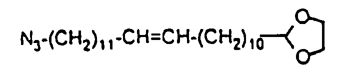

To 23-Chloro-1-(1,3-dioxalan-2-yl)tricosa-11-ene (7.7 g, 16.3 mmol) (synthesised through the Wittig coupling of 12-chlorododecanal and the triphenylphosphonium salt of its acetal using 1 equiv. of LDA in THF at 0°C) in DMF (120 cm³) was added sodium azide (7.4 g, 113.8 mmol) and the reaction stirred overnight under argon at ambient temperature. The solvent was reduced to approximately 20 cm³ and water (50 cm³) added. The solution was extracted with hexane, dried (MgSO₄) and the solvent removed to yield a waxy solid 7.023 g, 99%. δ_{H} (CDCl₃) 1.27 (32H, m, N₃(CH₂)₂(C*H*₂)₈CH₂CH=CHCH₂(C*H*₂)₈), 1.60 (4H, m, C*H*₂CH₂N₃, C*H*₂CHO), 2.00 (4H, m, C*H*₂CH=), 3.25 (2H, t, CH₂N₃), 3.83, 3.96 (4H, 2xm, OCH₂), 4.84 (1H, t, CHO), 5.34 (2H, m, CH=CH).

### (16) N-Azidotetracos-12-enyl(t-butyloxycarbonyl)aminopropyl-N,N'-bis(t-butyloxycarbonyl)-N'-t-butyloxycarbonylaminopropyl-1,4-diaminobutane

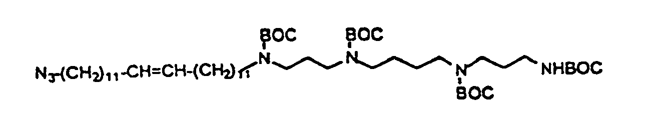

To a rapidly stirring suspension of silica (100 cm³) and 40% dichloromethane in hexane (100 cm³) was slowly added tosic acid (1 g) in water (3 cm³). The suspension was stirred for 10 minutes and used to pack a column. After washing the column with 40% dichloromethane in hexane 15 (537mg) was loaded and slowly eluted over 2 hours with 40% dichloromethane in hexane to give the aldehyde 464 mg, 96% as a colourless oil. To this aldehyde (464 mg, 1.185 mmol) and 9 (655 mg, 1.303 mmol) dissolved in anhydrous THF (40 cm³) were added Na₂SO₄, acetic acid (20 µl) and sodium triacetoxy borohydride (377 mg, 1.77 mmol) and the reaction left under argon overnight. All solids were filtered off, the solvent removed and the residues taken up into dichloromethane. The solution was washed (KOH_{(aq)}), dried (MgSO₄), and reduced in volume to approximately 30 cm³. To the solution was added tert-butyl dicarbonate (388 mg, 1.77 mmol) and the reaction left overnight. The solvent was removed and the residues purified by gradient silica chromatography (20-40% ethyl acetate in hexane) to give the desired product 352 mg, 30% as a colourless gum.

C₅₄H₁₀₃N₇O₈ requires 977.8. Found ES⁺: MH⁺, 978.7. δ_{H} (CDCl₃) 1.26 (34H. m, (C*H*₂)₈CH₂CH=CHCH₂(C*H*₂)₉), 1.44 (40H, m. (CH₃)₃C.

NCH₂(CH₂)₂CH₂N), 1.55-1.80 (6H, m, CH₂CH₂N₃, NCH₂C*H*₂CH₂N), 2.03 (4H, m, CH₂CH=), 3.05-3.40 (14H, m, NCH₂). 3.24 (2H, t, CH₂N₃), 5.34 (4H, m, CH=CH).

### (17) N-t-Butyloxycarbonyl-N-[t-butyloxcarbonylaminopropyl(t-butyloxycarbonyl)aminobutyl(t-butyloxycarbonyl)aminopropyl-1,24-diaminotetracosane

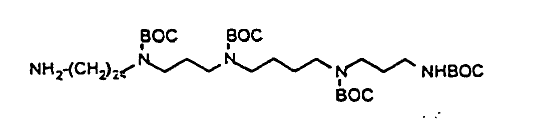

To 16 (204 mg) dissolved in t-butanol (20 cm³) at 40°C was added 10% palladium on carbon (40 and the atmosphere changed to hydrogen. The hydrogenation was maintained at 40°C for 3 days, the catalyst filtered off and the solvent removed. The residues were purified by silica column chromatography eluting with 10% methanol in dichloromethane to elute off impurities followed by 90:10:1 DCM:MeOH:NH₄OH to bring off the desired amine as a colourless gum 61mg,31%.

C₅₄H₁₀₇N₅O₈ requires 953.8. Found ES⁺: MH⁺, 954.6. δ_{H} (CDCl₃) 1.24 (42H, br, (C*H*₂)₂₁CH₂NH₂), 1.43 (42H, br, (CH₃)₃C, NCH₂(C*H*₂)₂CH₂N, C*H*₂(CH₂)₂₂NH₂), 1.70 (4H, m, NCH₂C*H*₂CH₂N), 2.70 (2H, t, C*H*₂NH₂), 3.0-3.4 (14H, brm, CH₂N), 5.35 (1H, b r, CONH).

### (18) N-t-Butyloxylarbonyl-N-[t-butyloxycarbonylaminopropyl(t-butyloxycarbonyl)aminobutyl(t-butyloxycarbonyl)aminopropyl-N'-glucuronyl-1,24-diaminotetracosane

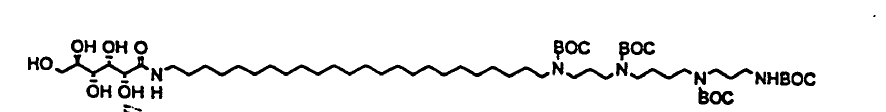

To 17 (62 mg, 0.065 mmol) in anhydrous methanol (8 cm³) were added δ-gluconolactone (17 mg, 0.097 mmol) and triethylamine (26 mg, 0.260 mmol) and the reaction stirred overnight at ambient temperature under argon. The solvents were removed and the residues purified by reverse phase chromatography eluting with 2:6:1 DCM:MeOH:H₂O to give the product, 32 mg, 43% as a white solid.

C₆₀H₁₁₇N₅O₁₄ requires 1131.9 Found ES⁺: MH⁺ , 1132.8. δ_{H} (CDCl₃) 1.25 (40H, br, N(CH₂)₂(C*H*₂)₂₀), 1.44 (40H, br, (CH₃)₃C, C*H*₂CH₂CO, NCH₂C*H*₂(CH₂)₂₀C*H*₂CH₂N), 1.6-1.8 (8H, m, NCH₂C*H*₂CH₂N, NCH₂(C*H*₂)₂CH₂N), 3.0-3.4 (16H, brm, NCH₂), 3.80 (4H, br, C*H*OH), 4.1, 4.3 (2H, 2xbr, C*H*₂OH), 5.37 (1H, br, NHCO₂), 7.22 (1H, br, NHCO).

### (19) N-Aminopropylaminobutylaminopropyl-N'-glucuronyl-1,24-diaminotetracosane

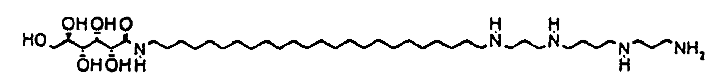

18 (32 mg) was treated as for the synthesis of 14 to give the product in quantitative yield as a white solid.

C₄₀H₈₅N₅O₆ requires 731.7 Found ES⁺: MH⁺ , 732.6, MH₂²⁺, 367.0. δ_{H} (CD₃OD) 1.28 (40H, br, NH(CH₂)₂(CH₂)₂₀), 1.53 (2H, p, CONHCH₂CH₂), 1.69 (2H, p, (C*H*₂)₂₂C*H*₂CH₂NHCH₂), 1.81 (4H, m, NCH₂(CH₂)₂CH₂N), 2.10 (4H, m, NCH₂CH₂CH₂N), 2.9-3.3 (16H, m. CH₂N), 3.55-3.85 (4H, m, CHOH), 4.09, 4.20 (2H, 2xm, C*H*₂OH).

### (20) 35-Azido-1-(1,3-dioxalan-2-yl)pentatriaconta-11,23-diene

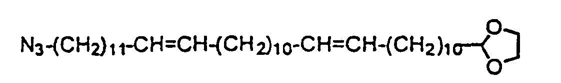

To 35-Chloro-1-(1,3-dioxalan-2-yl)pentatriaconta-11,23-diene (1.602 g, 2.690 mmol) (synthesised through the Wittig coupling of 12-chlorododecanal and the triphenylphosphonium salt of 23-Chloro-1-(1,3-dioxalan-2-yl)tricosa-11-ene using 1 equiv. of LDA in THF at 0°C) in anhydrous DMF (70 cm³) was added sodium azide (1.224 g, 18.83 mmol) and the reaction heated for 5 days at 50°C under argon. The solvent was reduced to almost dryness and the residues taken up into water (150 cm³) and ethyl acetate (150 cm³). The aqueous layer was extracted with ethyl acetate (4 x 150.cm³), the fractions combined, washed (2 x 150 cm³ water), dried (MgSO₄) and the solvent removed to quantitatively yield the azide as a pale yellow waxy solid.

C₃₈H₇₁N₃O2 requires 601.6. Found ES⁺: MNH₄⁺, 619.6. δ_{H} (CDCl₃) 1.27 (48H, br, N₃(CH₂)₂(C*H*₂)₈CH₂CH=CHCH₂(C*H*₂)₈CH₂CH=CHCH₂(C*H*₂)₈), 1.61 (4H, m, C*H*₂CH, C*H*₂CH₂N₃), 2.00 (8H, m, C*H*₂CH=), 3.25 (2H, t, CH₂N₃),. 3.8-4.05 (4H, m, CH₂O), 4.84 (1H, t, CH), 5.34 (4H, m, CH=CH).

### (21) N-Azidohexatriaconta-12,24-dienyl (t-butyloxycarbonyl) aminopropyl-N,N'-bis (t-butyloxycarbonyl)-N'-t-butyloxycarbonylaminopropyl-1,4-diaminobutane

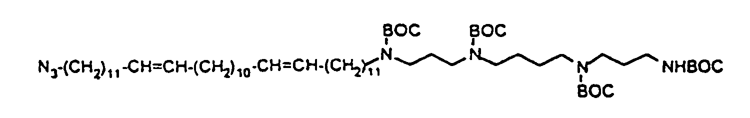

To a rapidly stirring suspension of silica (200 cm³) and 50% dichloromethane in hexane (200 cm³) was slowly added tosic acid (2 g) in water (6 cm³). The suspension was stirred for 10 minutes and used to pack a column. After washing the column with 50% dichloromethane in hexane 20 (998 mg) was loaded and eluted over 2 hours with 50% dichloromethane in hexane to give the aldehyde 591 mg, 65% as a colourless oil. To this aldehyde (591 mg, 1.059 mmol) and 9 (559 mg, 1.112 mmol) dissolved in anhydrous 30:70 MeOH:DCM (30 cm³) was added Na₂SO₄ and the reaction heated at reflux overnight. Sodium triacetoxy borohydride (449 mg, 2.118 mmol) was added and the reaction left for 4 hours. The drying agent was removed, water (1 cm³) added, and the reaction left overnight. t-Butyl dicarbonate (694 mg, 3.178 mmol) and triethylamine (536 mg, 5.296 mmol) were added and the reaction again left overnight. The solvent was removed and the residues purified by gradient silica chromatography eluting with 20-40% ethyl acetate in hexane to yield the product 226 mg, 19% as a colourless gum.

C₆₆H₁₂₅N₇O₈ requires 1144.0. Found ES⁺: MNa⁺ , 1166.8. δ_{H} (CDCl₃) 1.27 (50H, br, N₃CH₂(C*H*₂)₉CH₂CH=CHCH₂(C*H*₂)₈CH₂CH=CHCH₂(C*H*₂)₈), 1.4-1.9 (46H, m, (CH₃)₃C, C*H*₂CH₂N), 2.0 (8H, m, C*H*₂CH=), 3.05-3.4 (14H, m, CH₂N), 3.24 (2H, t, CH₂N₃), 5.34 (4H, m, CH=).

### (22) N-t-Butyloxycarbonyl-N-[t-butyloxycarbonylaminopropyl(t-butyloxycarbonyl)aminobutyl(t-butyloxycarbonyl)aminopropyl]-1,36-diaminohexatriacontane

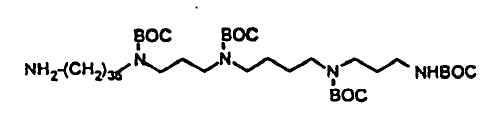

To 21 (220 mg) dissolved in tert-butanol at 40°C was added 10% palladium on carbon (60 mg) and the atmosphere changed to hydrogen. The hydrogenation was left for two days, the catalyst filtered off, the solvent removed and the residues purified by silica chromatography eluting with 100:10 dichloromethane : methanol then with 100:10:1 dichloromethane : methanol : NH₄OH to give the product as a colourless gum 147 mg, 68%.

C₆₆H₁₃₁N₅O₈ requires 1122.0 Found ES⁺: MH⁺ , 1122.9. δ_{H} (CDCl₃) 1.25 (64H, br s, (C*H*₂)₃₂(CH₂)₂NH₂), 1.4-1.8 (48H, br s, (CH₃)₃C, C*H*₂CH₂N), 2.88 (2H, t, C*H*₂NH₂), 3.0-3.4 (14H, br, CH₂N).

### (23) N-t-Butyloxycarbonyl-N-[t-butyloxycarbonylaminopropyl(t-butyloxycarbonyl)aminobutyl(t-butyloxycarbonyl)aminopropyl]-N'-glucuronyl-1,36-diaminohexatriacontane

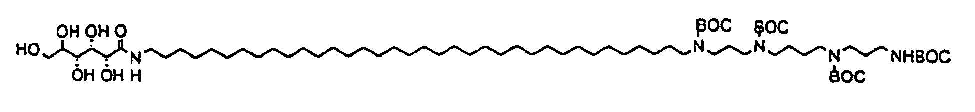

To 22 (130 mg, 0.116 mmol) in anhydrous methanol (8 cm³) under argon at 40°C were added triethylamine (47 mg, 0.463 mmol) and δ-gluconolactone (62 mg, 0.347 mmol) and the reaction left overnight. The solvent was removed and the residues purified by gradient reverse phase silica chromatography eluting with 2:6:1 to 2:6:0.25 dichloromethane : methanol : water to give the product as a colourless solid 83 mg, 55%.

C₇₂H₁₄N₅O₁₄ requires 1300.0 Found ES⁺: MNa⁺, 1323.0. δ_{H} (CDCl₃) 1.25 (64H, br, N(CH₂)₂(CH₂)₃₂), 1.35-1.80 (48H, br m, CH₂CH₂N, (CH₃)₃C), 3.0-3.4 (16H, br m, NCH₂), 3.55-4.0 (4H, br, CHOH), 4.20, 4.45 (2H, 2xbr, C*H*₂OH), 5.28 (1H, br, NHCO₂), 7.78 (1H, br, CONH(CH₂)₃₆).

### (24) N-aminopropylaminobutylaminopropyl-N'-glucuronyl-1,36-diaminohexatriacontane

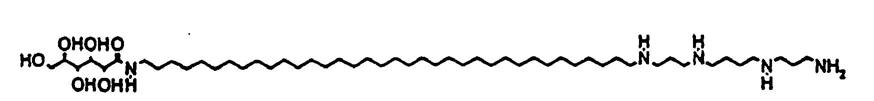

23 (83 mg) was treated as in the synthesis of 14 to quantitatively yield the product as a white solid.

C₅₂H₁₀₉₁N₅O₆ requires 899.8 Found ES⁺: MH⁺, 900.9. δ_{H} (CD₃OD) 0.8-2.2 (76H, br, (C*H*₂)₃₂(CH₂)₂N, C*H*₂CH₂N), 2.8-3.4 (16H, br, CH₂N), 3.5-3.9 (4H, br, C*H*OH), 4.08, 4.20 (2H, 2xbr, C*H*₂OH).

The advantageous properties of the lipids according to the invention may be demonstrated in the following tests:

### PRONASE and DNase treatment

One of the ultimate goals of current gene therapy research is to develop a delivery system which will remain stable and effective *in vivo,* since this would remove the need for expensive and time-consuming ex-vivo manipulations. Since intravenous administration offers the possibility of delivery to the largest number of tissue sites, survival of the gene delivery complex in the presence of serum could be an important feature of any effective technology. Many published studies have demonstated the susceptibility of gene delivery complexes to inactivation by serum even at levels as low as 10%. This effect is due, at least in part, to the destabilisation of complexes by a poorly understood mechanism, and this can lead to degradation of the DNA within the complex by serum-associated nucleases. We have therefore formed complexes between the lipids of the invention and plasmid DNA and subjected these to treatment with purified DNase and pronase as well as with 50% foetal calf serum. Integrity of the plasmid DNA was then measured.

### Methods

Plasmid DNA (pEG*lacZ*) was prepared at a concentration of 120µg/ml in water. A lipid according to the invention [for example the hexamine H18 described in the Example above] was prepared in a range of concentrations such that charge ratios of lipid:DNA of 0.25:1, 1:1, 2:1, 4:1, 8:1 would be obtained (based on the assumption that DNA at 120 µg/ml is equivalent to 0.387mM of negative charge, and the hexamine H18 for example at 10mg/ml is equivalent to 36.23mM of positive charge). An equal volume of DNA was added dropwise to a vortexing tube containing the lipid in water. For DNase treatment, DNase l (FPLCpure, Pharmacia) was added at a concentration of 1 unit/1µg of DNA and tubes were incubated at 37°C for 10 minutes. To inhibit further action of DNase, EGTA was added to a final concentration of 25mM. For pronase treatment, protease XIV (Sigma) was added to samples to a final concentration of 150 µg/ml, and samples incubated for 30 min at 37°C.

Complexes were disrupted in 0.5% SDS with incubation at 55°C for 20 min. Serum treatment involved incubating the samples in the presence of 50% foetal calf serum (final concentration) for 30 min at 37°C. EGTA was added to a final concentration of 50mM in an attempt to prevent subsequent action of serum-associated nucleases. Finally, all samples were analysed by electrophoresis on 0.8% agarose gels.

### Results

Analysis of the mobility of plasmid DNA through gels demonstrated that, as the amount of lipid increased. the DNA tended to be retarded in the wells. Thus, for example, at a charge ratio of 2:1 (H18:DNA), no DNA entered the gel, and at higher ratios, the plasmid DNA was no longer visible by ethidium bromide fluorescence (see below), suggesting that the DNA had become fully condensed. The H18/DNA condensates were resistant to treatment by pronase. In addition, DNA condensed with H18 at a charge ratio of at least 2:1 was resistant to treatment by DNase. At charge ratios of 2:1 or greater, addition of serum to 50% did not lead to an increase in the amount of DNA degradation, suggesting that lipids according to the invention are stable in serum.

### PHYSICAL CHEMICAL ASSAYS

Two physical chemical assays can be used to assess the ability of the lipids of the invention to compact supercoiled DNA and to determine the stability of the condensed particles.

### Assay 1

The first assay involves the use of ethidium bromide, a molecule which fluoresces when intercalated into the DNA helix. Solutions containing DNA and a lipid according to the invention are prepared so that the charge ratio between the negatively charged phosphate groups of the DNA and the postively charged polyamines of the lipids varies between zero and three [see the "Methods" in the previous section]. After ethidium bromide has been added to each solution the fluorescence reading is measured. As the charge ratio increases towards charge neutrality, because of the increasing amounts of lipid present, there is a progressive decrease in the fluorescence of the ethidium bromide when this molecule is excluded from binding to the DNA as compaction occurs. The point at which compaction is complete corresponds to the point at which the fluorescence reading levels-off at a minimum. In the case of thelipids of the invention the fluorescence minimum is reached at charge ratios of lipid to DNA in the range of 0.8 to 2.5. This assay has been used to demonstrate that lipid are compaction competent under conditions of physiological salt (150mM NaCl) and at acidic conditions down to pH 3.0. Repeating the assay also shows that the compacted DNA particles are stable for many hours both in physiological salt and under low pH conditions.

### Assay 2

The second assay involves gel electrophoresis using ethidium bromide as a stain. Samples of lipid and DNA are prepared as before and loaded into separate lanes in a polyacrylamide gel. After electrophoresis the fluorescence reading of each lane is determined. Two effects are observed. First, as the charge ratio increases towards neutrality the distance that the DNA/lipid complex travels through the gel decreases progressitvely. This is a result of two physical processes; compaction, which renders the DNA less able to move through the viscous gel and neutralisation of DNA negative charge, which reduces the electrostatic attraction between the complex and the cathode. Second, as the charge ratio increases beyond neutrality the brilliance of the fluorescent response decreases as the ethidium bromide stain is excluded from the DNA helix. This assay has been used to confirm that the lipids according to the invention cause DNA compaction close to the point of charge neutrality, in agreement with theory.

### TRANSFECTION OF MAMMALIAN CELLS WITH LIPID CONDENSED DNA COMPLEXES

### Condensation of DNA

Plasmid DNA (pEG/acZ) was prepared at concentrations of typically 60 or 120 µg/ml in water. Solutions of lipids according to the invention were prepared in water over a range of concentrations (typically 30 to 960 µg/ml). An equal volume of DNA was added dropwise to a tube containing a solution of the lipids whilst vortexing the tube.

### CHO Transfection protocol

Chinese Hamster Ovary (CHO) cells were seeded in to 24 well plates at 100,000 cells per well 24h before experiment. Cells were washed once in Optimem^{™} medium prior to transfection. Wash medium was removed and replaced with 0.5ml of Optimem^{™} to which the required amount of lipid condensed DNA was added (typically 1 to 5 µg DNA equivalent). Usually three replicate transfection wells were set up per condensed DNA sample tested. Cells were incubated for a further 3-4 h at 37°C, 5% CO₂ before removal of the complex and additon of 1ml of fresh medium (Iscoves^{™} medium: modified DMEM plus glutamate, asparagine, adenosine, guanosine, cytidine, uridine, thymidine and 10% dialysed foetal calf serum). Cells were cultured for a further 48-72 hours before harvesting and assay. Levels of Beta galactosidase reporter gene activity were determined using an enzyme assay system from Promega as follows. Cells were washed twice with 1 ml of phosphate buffered saline and solubilised in 200ul of 1 x cell lysis buffer. 50ul of cell extract was incubated with the provided buffer and substrate o-nitrophenyl-β-D-galactopyranoside and the optical density measured spectrophotometrically. Levels of β-gal expression were quantitated by reference to the standard curve and related to the amount of protein in the extract (measured using the BCA assay kit from Pierce) to give a final value expressed as mU of β-gal per mg of protein.

## Claims

1. A bipolar lipid comprising a cationic head (1), a hydrophobic backbone (2) and a hydrophilic tail (3) in which:
(A) the cationic head comprises two or more cationic centres, each centre containing at least one heteroatom which is capable of retaining a positive charge at a pH in the range 2-10 and which is covalently linked to the one or more other centres by one or more carbon-containing spacer groups;
(B) the hydrophobic backbone comprises at least one hydrocarbon chain; and
(C) the hydrophilic tail comprises at least one tail unit selected from synthetic polyols, naturally occurring polyols, poly(alkylene oxides) and derivatives thereof; each of said components (1) to (3) being covalently linked head (1) to backbone (2) to tail (3) and arranged such that said at least one hydrocarbon chain of the hydrophobic backbone (2) is covalently attached to one of said positive charge retaining heteroatoms in the cationic head (1) and to said at least one tail unit in the hydrophilic tail (3) such that the chain contains at least ten chain-linked atoms between its points of attachment to said positive chatge retaining heteroatom and to said at least one tail unit.

2. A lipid according to Claim 1 wherein each cationic centre is an amino group.

3. A lipid according to Claim 1 or Claim 2 wherein the number of cationic centres in the cationic head is from three to six.

4. A lipid according to any one of Claim 1 to Claim 3 wherein each carbon-containing spacer group in the cationic head is an optionally substituted aliphatic, cycloaliphatic, heteroaliphatic, heterocycloaliphatic, aromatic or heteroaromatic group.

5. A lipid according to any one of Claim 1 to Claim 4 wherein said at least one hydrocarbon chain in the hydrophobic backbone is an optionally substituted straight or branched aliphatic or heteroaliphatic chain containing from ten to around one hundred chain-linked atoms.

6. A lipid according to Claim 5 wherein the hydrophobic backbone has one or two hydrocarbon chains indirectly linked through a linker atom or group to one of said positive charge retaining heteroatoms in the cationic head (1).

7. A lipid according to any one of Claim 1 to Claim 5 wherein said at least one tail unit in the hydrophilic tail (3) is attached to a hydrocarbon chain of the hydrocarbon backbone (2) at the terminal carbon atom of said chain distal to the chain carbon atom attached to the cationic head (1).

8. A lipid according to any one of Claim 1 to Claim 7 which has the formula (1):
[R¹]ₘ- (L¹)ₙ-R² (1)
wherein R¹ is a hydrocarbon chain optionally substituted by one or more tail units selected from synthetic polyols, naturally occurring polyols, poly(alkylene oxides) and derivatives thereof, provided that at least one hydrocarbon chain R¹ is substituted by at least one such tail unit and that each such tail unit is attached to the hydrocarbon chain to achieve at least a ten chain-linked atom spacing along the chain between the tail unit and the group -(L¹)ₙ-R² ;
m is an integer from 1 to 6;
L¹ is a linker atom or group;
n is zero or the integer 1; and
R² is an optionally substituted aliphatic, cycloaliphatic, heteroaliphatic, heterocycloaliphatic, aromatic or heteroaromatic group containing two or more cationic centres, such that each [R¹]ₘ- (L¹)ₙ- group is attached to a positive charge retaining heteroatom in a cationic centre in R²;
and the salts, solvates and hydrates thereof.

9. A lipid according to Claim 8 wherein n in -(L¹)ₙ- is the integer 1.

10. A lipid according to Claim 9 wherein L¹ is a group -X¹Alk²- or - [X¹]₂Alk¹X¹Alk²- in which X¹ is an -O- or -S-atom or a -C(O) -, -C (O) O-, -C (S) -, -S (O) -, -S (O) ₂-, -N(R³) -, -CON(R³) -, -OC (O)N(R³) -, -CSN(R³) -, -N(R³) CO-, -N (R³) C (O) O-, -N (R³) CS-, -S(O)N(R³) -, -S(O)₂N(R³) -, -N(R³)S(O) -, -N(R³)S(O)₂-, -N(R³)CON(R³)- or -N(R³)SO₂N(R³)-group where each R³ is independently selected from hydrogen atoms, straight and branched alkyl groups and -Alk¹X¹- chains; and Alk¹ and Alk² may be the same or different and are each an optionally substituted straight or branched C₁₋₆ alkylene, C₂₋₆ alkenylene or C₂₋₆ alkynylene chain optionally interrupted or terminated by one or more carbocyclic or heterocarbocyclic groups, heteroatoms or heteroatom-containing groups X¹ as defined in this claim.

11. A lipid according to Claim 10 wherein X¹ is a -CONH- group, Alk¹ is a -CH₂-CH< chain and Alk² is a - (CH₂) ₄-, - (CH₂)₅- or - (CH2) ₆- chain.

12. A lipid according to Claim 8 which has the formula (1a):
[R⁷]ₚ-(L³)_{q}- [R⁶] ₘ- (L¹)ₙ-R² (1a)
wherein R², L¹, m and n are as defined for formula (1) ;
R⁶ is a hydrocarbon chain;
L³ is a linker atom or group;
R⁷ is a tail unit selected from synthetic polyols, naturally occurring polyols, poly(alkylene oxides) and derivatives thereof;
q is zero or an integer from one to six;
p is an integer from one to six;
provided that each R⁷ or L³ group, when present, is attached to a group R⁶ to achieve at least a ten chain-linked atom spacing along R⁶ between R⁷ or L³ and the group - (L¹) ₙ-R² ;
and the salts, solvates and hydrates thereof.

13. A lipid according to Claim 12 wherein R² is a group -WSp¹[WSp²]_{b}WSp³ or -WSp¹[WSp²]_{b}WH in which Sp¹, Sp² and Sp³, which may be the same or different, are each a spacer group, W is a cationic centre and b is zero or an integer from one to six.

14. A lipid according to Claim 13 wherein Sp¹, Sp² and Sp³ are each an optionally substituted aliphatic, cycloaliphatic, aromatic or heteroaromatic group.

15. A lipid according to Claim 14 wherein Sp¹, Sp² and Sp³ are each an optionally substituted C₁₋₆ alkylene chain.

16. A lipid according to any one of Claim 13 to Claim 15 wherein W is an -NH- group.

17. A lipid according to any one of Claim 13 to Claim 16 wherein b is an integer from 2 to 4.

18. A lipid according to Claim 12 wherein R² is a group -NH(Sp¹NHSp²] NH₂, -NH [Sp¹NHSp²NHSp²] NH₂ or -NH[Sp¹NHSp²NHSp²]NHCH₃ wherein Sp¹ is -CH₂- and each Sp² is -(CH₂)₃- or - -(CH₂)₄-.

19. A lipid according to any one of Claim 12 to Claim 18 wherein m is an integer 1 or 2.

20. A lipid according to any one of Claim 12 to Claim 19 wherein R⁶ is an optionally substituted C₁₀₋₆₀ aliphatic chain.

21. A lipid according to Claim 20 wherein R⁶ is a linear, optionally substituted C₁₆₋₃₈ alkylene chain.

22. A lipid according to any one of Claim 12 to Claim 21 wherein q is the integer 1 and p is an integer 1 or 2.

23. A lipid according to any one of Claim 12 to Claim 22 wherein L³ is an atom or group -X¹-, -X¹Alk¹X¹- or - [X¹Alk¹] ₂X¹Alk²X¹- in which X¹ is an -O- or -S- atom or a -C (O) -, -C (O) O-, -C(S)-, -S (O) -, -S(O)₂-, -N (R³) -, -CON(R³)-, -OC (O) N (R³) -, -CSN(R³)-, -N(R³)CO-, -N(R³)C(O)O-, -N(R³)CS-, -S (O) N (R³) -, -S(O)₂N(R³)-, -N(R³)S(O)-, -N(R³)S(O)₂-, -N(R³)CON(R³) - or -N(R³)SO₂N(R³)- group where each R³ is independently selected from hydrogen atoms, straight and branched alkyl groups and -Alk¹X¹- chains; and Alk¹ and Alk² may be the same or different and are each an optionally substituted straight or branched C₁₋₆ alkylene, C₂₋₆ alkenylene or C₂₋₆ alkynylene chain optionally interrupted or terminated by one or more carbocyclic or heterocarbocyclic groups, heteroatoms or heteroatom-containing groups X¹ as defined in this claim.

24. A lipid according to Claim 23 wherein L³ is a -NHCO-, -CONH-, -CONH (CH₂)₂ NHCO- or - [CONH (CH₂) ₂] ₂NCO (CH₂) ₂CONH- group.

25. A lipid according to any one of Claim 12 to Claim 24 wherein R⁷ is a poly(alkylene oxide) or a derivative thereof.

26. A lipid according to Claim 25 wherein R⁷ is a poly(ethylene oxide).

27. A lipid according to any one of Claim 12 to Claim 24 wherein R⁷ is a naturally occurring polyol.

28. A lipid according to claim 27 wherein R⁷ is an acyclic mono- or oligo-saccharide.

29. A targeted lipid composition comprising a bipolar lipid according to any one of Claim 1 to Claim 28 assembled with at least one targeting molecule.

30. A composition according to Claim 29 wherein said at least one targeting molecule is an antibody or a fragment or a derivative thereof.

31. A lipid complex comprising a bipolar lipid according to any one of Claim 1 to Claim 28 in association with at least one bioactive substance.

32. A complex according to Claim 31 wherein said at least one bioactive substance is a bioactive protein, peptide, polysaccharide, nucleic acid, oligonucleotide or derivative thereof, lipid, glycolipid, lipoprotein, lipopolysaccharide or viral, bacterial, protozoal, cellular or tissue fraction.

33. A complex according to Claim 32 wherein said at least one bioactive substance is a polyanion.

34. A complex according to Claim 33 wherein said at least one bioactive substance is a nucleic acid.

35. A complex according to any one of Claim 31 to Claim 34 containing two or more different bipolar lipids.

36. A complex according to Claim 35 wherein one bipolar lipid has tail units formed by a poly(alkylene oxide) or a derivative thereof and the other or others has or have tail units formed by a synthetic or naturally occurring polyol.

37. A complex according to Claim 36 wherein the poly(alkylene oxide) is poly(ethylene oxide).

38. A complex according to any one of Claim 31 to Claim 37 additionally comprising at least one targeting molecule.

39. A complex according to Claim 38 wherein the bipolar lipid is associated with said at least one targeting molecule.

40. A composition comprising a complex according to any one of Claim 31 to Claim 39 and at least one other lipid.

41. A composition according to Claim 40 wherein said at least one other lipid is a neutral or cationic lipid.

42. A composition comprising a complex according to any one of Claim 31 to Claim 41 and one or more pharmaceutically acceptable carriers, excipients or diluents.

43. A method of preparing a bipolar lipid as defined in any one of Claim 1 to Claim 28 comprising coupling:
(A) a cationic head comprising two or more cationic centres, each centre containing at least one heteroatom which is capable of retaining a positive charge at a pH in the range 2-10 and which is covalently linked to the one or more other centres by one or more carbon-containing spacer groups;
(B) a hydrophobic backbone comprising at least one hydrocarbon chain; and
(C) a hydrophilic tail comprising at least one tail unit selected from synthetic polyols, naturally occurring polyols, poly(alkylene oxides) and derivatives thereof, wherein starting materials (A), (B) and (C) contain one or more reactive functional groups suitable for facilitating coupling such that said at least one hydrocarbon chain of the hydrophobic backbone is covalently attached to one of said positive charge retaining heteroatoms in the cationic head and to said at least one tail unit in the hydrophilic tail such that the chain contains at least ten chain-linked atoms between its points of attachments to said positive charge retaining heteroatom and to said at least one tail unit.

44. A method according to Claim 43 comprising the step of deprotecting a protected derivative of said lipid.

45. A method of preparing a lipid complex as defined in any one of Claim 31 to Claim 39 comprising mixing said bipolar lipid with a bioactive substance.

46. Use of a complex as defined in any one of Claim 31 to Claim 39 for delivering a bioactive substance to cells *in vitro.*

47. Use of a composition as defined in any one of Claim 40 to Claim 42 for the preparation of a medicament for the delivery of a bioactive substance.

48. Use as claimed in Claim 46 or Claim 47 wherein said bioactive substance is a therapeutic, diagnostic or immunomodulating agent.

## Patentansprüche

1. Bipolares Lipid, umfassend einen kationischen Kopf (1), ein hydrophobes Gerüst (2) und einen hydrophilen Schwanz (3), worin:
(A) der kationische Kopf zwei oder mehrere kationische Zentren umfasst, wobei jedes Zentrum wenigstens ein zur Ausbildung einer positiven Ladung bei einem pH im Bereich von 2 bis 10 fähiges Heteroatom enthält, welches über eine oder mehrere kohlenstoffhaltige Spacergruppen kovalent an ein oder mehrere andere Zentren gebunden ist;
(B) das hydrophobe Gerüst wenigstens eine Kohlenwasserstoffkette umfasst; und
(C) der hydrophile Schwanz wenigstens eine Schwanzeinheit umfasst, die unter synthetischen Polyolen, natürlich vorkommenden Polyolen, Poly(alkylenoxiden) und Derivaten davon ausgewählt ist; wobei jede Komponente (1) bis (3) Kopf (1) an Gerüst (2) an Schwanz (3) kovalent gebunden und so angeordnet ist, dass die wenigstens eine Kohlenwasserstoffkette des hydrophoben Gerüsts (2) kovalent an eines der die positive Ladung aufnehmenden Heteroatome im kationischen Kopf (1) und an die wenigstens eine Schwanzeinheit im hydrophilen Schwanz (3) gebunden ist, so dass die Kette wenigstens 10 kettenverbundene Atome zwischen den Anbindungspunkten zum die positive Ladung aufnehmenden Heteroatom und die wenigstens eine Schwanzeinheit enthält.

2. Lipid nach Anspruch 1, wobei jedes kationische Zentrum eine Aminogruppe ist.

3. Lipid nach Anspruch 1 oder Anspruch 2, wobei die Zahl der kationischen Zentren im kationischen Kopf drei bis sechs beträgt

4. Lipid nach einem der Ansprüche 1 bis 3, wobei jede Kohlenstoff enthaltende Spacergruppe im kationischen Kopf eine gegebenenfalls substituierte aliphatische, cycloaliphatische, heteroaliphatische, heterocycloaliphatische, aromatische oder heteroaromatische Gruppe ist.

5. Lipid nach einem der Ansprüche 1 bis 4, wobei die wenigstens eine Kohlenwasserstoffkette im hydrophoben Gerüst eine gegebenenfalls substituierte geradkettige oder verzweigte aliphatische oder heteroaliphatische Kette mit 10 bis etwa 100 kettenverbundenen Atomen ist.

6. Lipid nach Anspruch 5, wobei das hydrophobe Gerüst eine oder zwei Kohlenwasserstoffketten aufweist, die über ein Linkeratom oder -gruppe indirekt an eines der die positive Ladung aufnehmenden Heteroatome im kationischen Kopf (1) gebunden sind.

7. Lipid nach einem der Ansprüche 1 bis 5, wobei die wenigstens eine Schwanzeinheit im hydrophilen Schwanz (3) am terminalen Kohlenstoffatom einer Kohlenwasserstoffkette des Kohlenwasserstoffgerüsts (2) an die Kette gebunden ist, das sich distal zum Kettenkohlenstoffatom befindet, das an den kationischen Kopf (1) gebunden ist.

8. Lipid nach einem der Ansprüche 1 bis 7, mit der Formel (1):
[R¹]ₘ-(L¹)ₙ-R² (1)
worin R¹ für eine Kohlenwasserstoffkette steht, die gegebenenfalls mit einer oder mehreren Schwanzeinheiten substituiert ist, die unter synthetischen Polyolen, natürlich vorkommenden Polyolen, Poly(alkylenoxiden) und Derivaten davon ausgewählt ist, mit der Maßgabe, dass wenigstens eine Kohlenwasserstoffkette R¹ durch wenigstens eine derartige Schwanzeinheit substituiert ist und dass jede derartige Schwanzeinheit so an die Kohlenwasserstoffkette gebunden ist, dass ein Abstand von wenigstens 10 kettenverbundenen Atomen entlang der Kette zwischen der Schwanzeinheit und der Gruppe
-(L¹)ₙ-R² erreicht wird;
m für eine ganze Zahl von 1 bis 6 steht;
L¹ für ein Linkeratom oder-gruppe steht;
n für Null oder die ganze Zahl 1 steht; und
R² für eine gegebenenfalls substituierte aliphatische, cycloaliphatische, heteroaliphatische, heterocycloaliphatische, aromatische oder heteroaromatishe Gruppe steht, die zwei oder mehr kationische Zentren enthält, so dass jede [R¹]ₘ-(L¹)ₙ-Gruppe an ein eine positive Ladung aufnehmendes Heteroatom in einem kationischen Zentrum in R² gebunden ist;
und die Salze, Solvate und Hydrate davon.

9. Lipid nach Anspruch 8, worin n in -(L¹)ₙ- für de ganze Zahl 1 steht.

10. Lipid nach Anspruch 9, worin L¹ für eine Gruppe -X¹Alk²- oder -[X¹]₂Alk¹X¹Alk²-, in welcher X¹ für ein -O- oder -S-Atom oder eine -C(O)-, -C(O)O-, -C(S)-, -S(O)-, -S(O)₂-, -N(R³), -CON(R³)-, -OC(O)N(R³)-, -CSN(R³)-, -N(R³)CO-, -N(R³)C(O)O-, -N(R³)CS-. -S(O)N(R³)-, -S(O)₂N(R³)-, -N(R³)S(O)-, -N(R³)S(O)₂-, -N(R³)CON(R³)- oder -N(R³)SO₂N(R³)-Gruppe steht, worin jedes R³ unabhängig ausgewählt ist unter Wasserstoffatomen, geradkettigen und verzweigten Alkylgruppen und -Alk¹X¹-Ketten; und Alk¹ und Alk² gleich oder verschieden sein können und jeweils für eine gegebenenfalls substituierte geradkettige oder verzweigte C₁₋₆-Alkylen-, C₂₋₆-Alkenylen oder C₂₋₆-Alkinylen-Kette stehen, die gegebenenfalls durch eine oder mehrere carbocyclische oder heterocarbocyclische Gruppen, Heteroatome oder heteroatomhaltige Gruppen X¹ gemäß Definition in diesem Anspruch unterbrochen oder terminiert ist.

11. Lipid nach Anspruch 10, worin X¹ für eine -CONH-Gruppe steht, Alk¹ für eine -CH₂-CH< Kette und Alk² für eine -(CH₂)₄-, -(CH₂)₅ oder (CH₂)₆-Kette steht.

12. Lipid nach Anspruch 8 mit der Formel (1a):
[R⁷]ₚ-(L³)_{q}-[R⁶]ₘ-L¹)ₙ-R² (1a)
worin R², L¹, m und n die für die Formel (1) angegebene Bedeutung haben;
R⁶ für eine Kohlenwasserstoffkette steht;
L³ für ein Linkeratom oder -gruppe steht;
R⁷ für eine Schwanzgruppe steht, die ausgewählt ist unter synthetischen Polyolen, natürlich vorkommenden Polyolen, Poly(alkylenoxiden) und Derivaten davon;
q für Null oder eine ganze Zahl von eins bis sechs steht;
p für eine ganze Zahl von eins bis sechs steht;
mit der Maßgabe, dass jede Gruppe R⁷ oder L³, sofern vorliegend, so an eine Gruppe R⁶gebunden ist, dass ein Abstand von wenigstens zehn kettenverbundenen Atomen entlang R⁶ zwischen R⁷ oder L³ und der Gruppe -(L¹)ₙ-R² erreicht wird;
und die Salze, Solvate und Hydrate davon.

13. Lipid nach Anspruch 12, worin R² für eine Gruppe -WSp¹[WSp²]_{b}WSp³ oder -WSp¹[WSp²]_{b}WH steht, worin Sp¹, Sp² und Sp³, die gleich oder verschieden sein können, jeweils für eine Spacergruppe stehen, W für ein kationisches Zentrum steht und b für Null oder eine ganze Zahl von 1 bis 6 steht.

14. Lipid nach Anspruch 13, worin Sp¹, Sp² und Sp³ jeweils für eine gegebenenfalls substituierte aliphatische, cycloaliphatische, aromatische oder heteroaromatische Gruppe stehen.

15. Lipid nach Anspruch 14, worin Sp¹, Sp² und Sp³ jeweils für eine gegebenenfalls substituierte C₁₋₆-Alkylenkette stehen.

16. Lipid nach einem der Ansprüche 13 bis 15, worin W für eine -NH-Gruppe steht.

17. Lipid nach einem der Ansprüche 13 bis 16, worin b für eine ganze Zahl von 2 bis 4 steht.

18. Lipid nach Anspruch 12, worin R² für eine Gruppe -NH[Sp¹NHSp]NH₂, -NH[Sp¹NHSp²NHSp²]NH₂, oder -NH[Sp¹NHSp² NHSp²]NHCH₃ steht, worin Sp¹ für -CH₂- und jedes Sp² für -(CH₂)₃- oder -(CH₂)₄- steht.

19. Lipid nach einem der Ansprüche 12 bis 18, worin m für eine ganze Zahl 1 oder 2 steht.

20. Lipid nach einem der Ansprüche 12 bis 19, worin R⁶ für eine gegebenenfalls substituierte aliphatische C₁₀₋₆₀-Kette steht.

21. Lipid nach Anspruch 20, worin R⁶ für eine lineare, gegebenenfalls substituierte C₁₆₋₃₆-Alkylenkette steht.

22. Lipid nach einem der Ansprüche 12 bis 21, worin q für die ganze Zahl 1 steht und p für eine ganze Zahl 1 oder 2 steht.

23. Lipid nach einem der Ansprüche 12 bis 22, worin L³ für ein Atom oder Gruppe -X¹-, -X¹Alk¹ X¹- oder -[X¹Alk¹X¹Alk²X¹- steht, worin X¹ für ein -O- oder -S-Atom oder eine -C(O)-, -C(O)O-, -C(S)-, -S(O)-, -S(O)₂-, -N(R³), -CON(R³)-, -OC(O)N(R³)-, -CSN(R³)-, -N(R³)CO-, -N(R³)C(O)O-, -N(R³)CS-, -S(O)N(R³)-, -S(O)₂N(R3)-, -N(R³)S(O), -N(R³)S(O)₂-, -N(R³)CON(R³)- oder -N(R³)SO₂N(R³)-Gruppe steht, worin jedes R³ unabhängig ausgewählt ist unter Wasserstoffatomen, geradkettigen und verzweigten Alkylgruppen und -Alk¹X¹-Ketten; und Alk¹ und Alk² gleich oder verschieden sein können und jeweils für eine gegebenenfalls substituierte geradkettige oder verzweigte C₁₋₆-Alkylen-, C₂₋₆-Alkenylen- oder C₂₋₆-Alkinylenkette stehen, die gegebenenfalls durch eine oder mehrere carbocyclische oder heterocarbocyclische Gruppen, Heteroatome oder heteroatomhaltige Gruppen X¹ gemäß Definition in diesem Anspruch unterbrochen oder terminiert ist.

24. Lipid nach Anspruch 23, worin L³ für eine -NHCO-, -CONH-, -CONH(CH₂)₂NHCO- oder -[CONH(CH₂)₂]₂NCO(CH₂)₂CONH-Gruppe steht.

25. Lipid nach einem der Ansprüche 12 bis 24, worin R⁷ ein Poly(alkylenoxid) oder ein Derivat davon ist.

26. Lipid nach Anspruch 25, worin R⁷ ein Poly(ethylenoxid) ist.

27. Lipid nach einem der Ansprüche 12 bis 24, worin R⁷ ein natürlich vorkommendes Polyol ist.

28. Lipid nach Anspruch 27, worin R⁷ ein acylisches Mono- oder Oligosaccharid ist.

29. Wirkort-gerichtete Lipidzusammensetzung, umfassend ein bipolares Lipid nach einem der Ansprüche 1 bis 28 zusammen mit wenigstens einem dirigierenden Molekül.

30. Zusammensetzung nach Anspruch 29, worin das wenigstens eine dirigierende Molekül ein Antikörper oder ein Fragment oder ein Derivat davon ist.

31. Lipidkomplex, umfassend ein bipolares Lipid nach einem der Ansprüche 1 bis 28, assoziiert mit wenigstens einer bioaktiven Substanz.

32. Komplex nach Anspruch 31, worin die wenigstens eine bioaktive Substanz, ein bioaktives Protein, Peptid, Polysaccharid, Nukleinsäure, Oligonukleotid oder Derivat davon, Lipid, Glycolipid, Lipoprotein, Lipopolysaccharid oder eine virale, bakterielle, protozoale, zelluläre oder Gewebefraktion ist.

33. Komplex nach Anspruch 32, worin die wenigstens eine bioaktive Substanz ein Polyanidn ist.

34. Komplex nach Anspruch 33, worin die wenigstens eine bioaktive Substanz eine Nukleinsäure ist.

35. Komplex nach einem der Ansprüche 31 bis 34, enthaltend zwei oder mehrere unterschiedliche bipolare Lipide.

36. Komplex nach Anspruch 35, worin ein bipolares Lipid eine von einem Poly(alkylenoxid) oder einem Derivat davon ausgebildete Schwanzeinheiten aufweist und das oder die anderen von einem synthetischen oder natürlich vorkommenden Polyol ausgebildete Schwanzeinheiten aufweist (aufweisen).

37. Komplex nach Anspruch 36, worin das Poly(alkylenoxid) Poly(ethylenoxid) ist.

38. Komplex nach einem der Ansprüche 31 bis 37, zusätzlich umfassend wenigstens ein dirigierendes Molekül.

39. Komplex nach Anspruch 38, worin das bipolare Lipid mit dem wenigstens einen dirigierenden Molekül assoziiert ist.

40. Zusammensetzung, umfassend einen Komplex nach einem der Ansprüche 31 bis 39 und wenigstens ein weiteres Lipid.

41. Zusammensetzung nach Anspruch 40, worin das wenigstens eine weitere Peptid ein neutrales oder kationisches Lipid ist.

42. Zusammensetzung, umfassend einen Komplex nach einem der Ansprüche 31 bis 41 und pharmazeutisch akzeptable Träger, Exzipienten und/oder Verdünnungsmittel.

43. Verfahren zur Herstellung eines bipolaren Lipids gemäß Definition in einem der Ansprüche 1 bis 28, bei dem man kuppelt:
(A) einen kationischen Kopf (2), der zwei oder mehrere kationische Zentren umfasst, wobei jedes Zentrum wenigstens ein zur Ausbildung einer positiven Ladung bei einem pH im Bereich von 2 bis 10 fähiges Heteroatom enthält, welches über eine oder mehrere kohlenstoffhaltige Spacergruppen kovalent an ein oder mehrer andere Zentren gebunden sind;
(B) ein hydrophobes Gerüst, das wenigstens eine Kohlenwasserstoffkette umfasst; und
(C) einen hydrophilen Schwanz, der wenigstens eine Schwanzeinheit umfasst, die unter synthetischen Polyolen, natürlich vorkommenden Polyolen, Poly(alkylenoxiden) und Derivaten davon ausgewählt ist; wobei die Ausgangsmaterialien (A), (B) und (C), eine oder mehrere funktionelle Gruppen aufweisen, die zur Ermöglichung der Kupplung geeignet sind, dass die wenigstens eine Kohlenwasserstoffkette des hydrophoben Gerüsts kovalent an eines der die positive Ladung aufnehmenden Heteroatome im kationischen Kopf und an die wenigstens eine Schwanzeinheit im hydrophilen Schwanz gebunden ist, so dass die Kette wenigstens 10 kettenverbundene Atome zwischen den Anbindungspunkten zum die positive Ladung aufnehmenden Heteroatom und die wenigstens eine Schwanzeinheit enthält.

44. Verfahren nach Anspruch 43, bei dem man ein geschütztes Derivat des Lipids entschützt.

45. Verfahren zur Herstellung eines Lipidkomplexes gemäß Definition in einem der Ansprüche 31 bis 39, bei dem man das bipolare Lipid mit einer bioaktiven Substanz vermischt.

46. Verwendung eines Komplexes gemäß Definition in einem der Ansprüche 31 bis 39 zum *in vitro*-Transport einer bioaktiven Substanz zu Zellen.

47. Verwendung einer Zusammensetzung gemäß Definition in einem der Ansprüche 40 bis 42 zur Herstellung eines Arzneimittels zum Transport einer bioaktiven Substanz.

48. Verwendung nach Anspruch 46 oder Anspruch 47, wobei die bioaktive Substanz ein therapeutisches, diagnostisches oder immunmodulierendes Mittel ist.

## Revendications

1. Lipide bipolaire comprenant une tête cationique (1), un squelette hydrophobe (2) et une queue hydrophile (3), dans lequel :
(A) la tête cationique comprend deux centres cationiques ou plus, chaque centre contenant au moins un hétéroatome qui est capable de conserver une charge positive à un pH dans la gamme de 2 à 10 et qui est lié de manière covalente au un ou plusieurs autres centres par un ou plusieurs groupes espaceurs contenant des atomes de carbone ;
(B) le squelette hydrophobe comprend au moins une chaîne hydrocarbonée ; et
(C) la queue hydrophile comprend au moins un motif de queue choisi parmi les polyols synthétiques, les polyols d'origine naturelle, les poly(oxydes d'alkylène) et leurs dérivés ; chacun desdits composants (1) à (3) étant une tête (1) liée de manière covalente à un squelette (2) lié de manière covalente à une queue (3) et disposé de sorte que ladite au moins une chaîne hydrocarbonée du squelette hydrophobe (2) est liée de manière covalente à un desdits hétéroatomes conservant une charge positive dans la tête cationique (1) et audit au moins un motif de queue dans la queue hydrophile (3) de sorte que la chaîne contient au moins dix atomes liés en une chaîne entre ses points de liaison audit hétéroatome conservant une charge positive et audit au moins un motif de queue.

2. Lipide selon la revendication 1, dans lequel chaque centre cationique est un groupe amino.

3. Lipide selon la revendication 1 ou la revendication 2, dans lequel le nombre de centres cationiques dans la tête cationique est de trois à six.

4. Lipide selon l'une quelconque des revendications 1 à 3, dans lequel chaque groupe espaceur contenant des atomes de carbone dans la tête cationique est un groupe aliphatique, cycloaliphatique, hétéroaliphatique, hétérocycloaliphatique, aromatique ou hétéroaromatique éventuellement substitué.

5. Lipide selon l'une quelconque des revendications 1 à 4, dans lequel ladite au moins une chaîne hydrocarbonée dans le squelette hydrophobe est une chaîne aliphatique ou hétéroaliphatique linéaire ou ramifiée éventuellement substituée contenant de dix à environ cent atomes liés en une chaîne.

6. Lipide selon la revendication 5, dans lequel le squelette hydrophobe contient une ou deux chaînes hydrocarbonées indirectement liées par un atome ou un groupe de liaison à l'un desdits hétéroatomes conservant une charge positive dans la tête cationique (1).

7. Lipide selon l'une quelconque des revendications 1 à 5, dans lequel ledit au moins un motif de queue dans la queue hydrophile (3) est lié à une chaîne hydrocarbonée du squelette hydrocarboné (2) à l'atome de carbone terminal de ladite chaîne distal de l'atome de carbone de la chaîne liée à la tête cationique (1).

8. Lipide selon l'une quelconque des revendications 1 à 7 qui a la formule (1) :
[R¹]m-(L¹)ₙ-R² (1)
dans laquelle
R¹ représente une chaîne hydrocarbonée éventuellement substituée par un ou plusieurs motifs de queue choisis parmi les polyols synthétiques, les polyols d'origine naturelle, les poly(oxydes d'alkylène) et leurs dérivés, à condition qu'au moins une chaîne hydrocarbonée R¹ soit substituée par au moins un de ces motifs de queue et que chacun de ces motifs de queue soit lié à la chaîne hydrocarbonée pour obtenir un espacement d'au moins dix atomes liés en une chaîne le long de la chaîne entre le motif de queue et le groupe -(L¹)ₙ-R²;
m représente un nombre entier de 1 à 6 ;
L¹ représente un atome ou un groupe de liaison;
n représente zéro ou le nombre entier 1 ; et
R² représente un groupe aliphatique, cycloaliphatique, hétéroaliphatique, hétérocycloaliphatique, aromatique ou hétéroaromatique éventuellement substitué contenant deux centres cationiques ou plus, de sorte que chaque groupe (R¹)ₘ-(L¹)ₙ- est lié à un hétéroatome conservant une charge positive dans un centre cationique en R² ;
et ses sels, solvates et hydrates.

9. Lipide selon la revendication 8, dans lequel n dans le groupe -(L¹)ₙ- représente le nombre entier 1.

10. Lipide selon la revendication 9, dans lequel L¹ est un groupe -X¹Alk²- ou -[X¹]₂Alk¹X¹Alk₂-, dans lequel X¹ représente un atome -O- ou -S- ou un groupe -C(O)-, -C(O)O-, -C(S)-, -S(O)-, -S(O)₂-, -N(R³)-, -CON(R³)-, -OC(O)N(R³)-, -CSN(R³)-, -N(R³)CO-, -N(R³)C(O)O-, -N(R³)CS-, -S(O)N(R³)-, -S(O)₂N (R³)-, -N(R³)S(O)-, -N(R³)S(O)₂-, -N(R³)CON(R³)- ou -N(R³)SO₂N(R³)-, où chaque R³ est indépendamment choisi parmi un atome d'hydrogène, un groupe alkyle linéaire ou ramifié et une chaîne -Alk¹X¹- ; et Alk¹ et Alk² peuvent être identiques ou différents et représentent chacun une chaîne alkylène en C₁ à C₆, alcénylène en C₂ à C₆ ou alcynylène en C₂ à C₆ linéaire ou ramifiée éventuellement substituée et éventuellement interrompue ou terminée par un ou plusieurs groupes carbocycliques ou hétérocarboèyc1iques, hétéroatomes ou groupes X¹ contenant des hétéroatomes tels que définis dans la présente revendication.

11. Lipide selon la revendication 10, dans lequel X¹ représente un groupe -CONH-, AlK¹ représente une chaîne -CH₂-CH< et Alk² représente une chaîne -(CH₂)₄-, -(CH₂)5- ou -(CH₂)₆-.

12. Lipide selon la revendication 8 qui a la formule (1a) :
[R¹]ₚ-(L³)_{q}-[R⁶]ₘ-(L¹)ₙ-R² (1a)
dans laquelle
R², L¹, m et n sont tels que définis pour la formule (1) ;
R⁶ représente une chaîne hydrocarbonée ;
L³ représente un atome ou un groupe de liaison ;
R⁷ représente un motif de queue choisi parmi les polyols synthétiques, les polyols d'origine naturelle, les poly(oxydes d'alkylène) et leurs dérivés ;
q représente zéro ou un nombre entier de un à six ;
p représente un nombre entier de un à six ;
à condition que chaque groupe R¹ ou L³, si présent, soit lié à un groupe R⁶ pour obtenir un espacement d'au moins dix atomes liés en une chaîne le long de R⁶ entre R¹ ou L³ et le groupe -(L¹)-R₂ ;
et ses sels, solvates et hydrates.

13. Lipide selon la revendication 12, dans lequel R² représente un groupe -WSp¹[WSp²]_{b}WSp³ ou -WSp¹[WSp²]_{b}WH, où Sp¹, Sp² et Sp³, qui peuvent être identiques ou différents, représentent chacun un groupe espaceur, W représente un centre cationique et b représente zéro ou un nombre entier de un à six.

14. Lipide selon la revendication 13, dans lequel Sp¹, Sp² et Sp³ représentent chacun un groupe aliphatique, cycloaliphatique, aromatique ou hétéroaromatique éventuellement substitué.

15. Lipide selon la revendication 14, dans lequel Sp¹, Sp² et Sp³ représentent chacun une chaîne alkylène en C₁ à C₆ éventuellement substituée.

16. Lipide selon l'une quelconque des revendications 13 à 15, dans lequel W représente un groupe -NH-.

17. Lipide selon l'une quelconque des revendications 13 à 16, dans lequel b représente un nombre entier de 2 à 4.

18. Lipide selon la revendication 12, dans lequel R² représente un groupe -NH[Sp¹NHSp²]NH₂, -NH[Sp¹NHSp²NHSp²]NH₂ ou -NH(Sp¹NHSp²NHSp²]NHCH₃, où Sp¹ représente un groupe -CH₂- et chaque Sp² représente un groupe -(CH₂)₃- ou -(CH₂)₄-.

19. Lipide selon l'une quelconque des revendications 12 à 18, dans lequel m représente le nombre entier 1 ou 2.

20. Lipide selon l'une quelconque des revendications 12 à 19, dans lequel R⁶ représente une chaîne aliphatique en C₁₀ à C₆₀ éventuellement substituée.

21. Lipide selon la revendication 20, dans lequel R⁶ représente une chaîne alkylène linéaire en C₁₆ à C₃₈ éventuellement substituée.

22. Lipide selon l'une quelconque des revendications 12 à 21, dans lequel q représente le nombre entier 1 et p représente le nombre entier 1 ou 2.

23. Lipide selon l'une quelconque des revendications 12 à 22, dans lequel L³ représente un atome ou un groupe -X¹-, -X¹Alk¹X¹- ou -[X¹Alk¹]₂X¹Alk²X¹-, où X¹ représente un atome -O- ou -S- ou un groupe -C(O)-, -C(O)O-, -C(S)-, -S(O)-, -S(O)₂-, -N(R³)-, -CON(R³)-, -OC(O)N(R³)-, -CSN(R³)-, -N(R³)CO-, -N(R³)C(O)O-, -N(R³)CS-, -S(O)N(R³)-, -S(O)₂N(R³)-, -N(R³)S(O)-, -N(R³)S(O)₂-, -N(R³)CON(R³)- ou -N(R³)SO₂N (R³)-, où chaque R³ est indépendamment choisi parmi un atome d'hydrogène, un groupe alkyle linéaire ou ramifié et une chaîne -Alk¹X¹- ; et Alk¹ et Alk² peuvent être identiques ou différents et représentent chacun une chaîne alkylène en C₁ à C₆, alcénylène en C₂ à C₆ ou alcynylène en C₂ à C₆ linéaire ou ramifiée éventuellement substituée, et éventuellement interrompue ou terminée par un ou plusieurs groupes carbocycliques ou hétérocarbocycliques, hétéroatomes ou groupes X¹ contenant des hétéroatomes tels que définis dans la présente revendication.

24. Lipide selon la revendication 23, dans lequel L³ représente un groupe -NHCO-, -CONH-, -CONH(CH₂)₂NHCO- ou -[CONH(CH₂)₂]₂NCO(CH₂)₂CONH-.

25. Lipide selon l'une quelconque des revendications 12 à 24, dans lequel R⁷ représente un poly(oxyde d'alkylène) ou un de ses dérivés.

26. Lipide selon la revendication 25, dans lequel R⁷ représente un poly(oxyde d'éthylène).

27. Lipide selon l'une quelconque des revendications 12 à 24, dans lequel R⁷ est un polyol d'origine naturelle.

28. Lipide selon la revendication 27, dans lequel R⁷ représente un mono- ou oligo-saccharide acyclique.

29. Composition de lipide ciblée comprenant un lipide bipolaire selon l'une quelconque des revendications 1 à 28 associé à au moins une molécule de ciblage.

30. Composition selon la revendication 29, dans lequel ladite au moins une molécule de ciblage est un anticorps ou un fragment ou un dérivé de celui-ci.

31. Complexe lipidique comprenant un lipide bipolaire selon l'une quelconque des revendications 1 à 28 associé à au moins une substance bioactive.

32. Complexe selon la revendication 31, dans lequel ladite au moins une substance bioactive est une protéine, un peptide, un polysaccharide, un acide nucléique, un oligonueléotide ou un de ses dérivés, un lipide, un glycolipide, une lipoprotéine, un lipopolysaccharide, tous bioactifs, ou toute fraction virale, bactérienne, protozoaire, cellulaire ou tissulaire.

33. Complexe selon la revendication 32, dans lequel ladite au moins une substance bioactive est un polyanion.

34. Complexe selon la revendication 33, dans lequel ladite au moins une substance bioactive est un acide nucléique.

35. Complexe selon l'une quelconque des revendications 31 à 34 contenant deux lipides bipolaires différents, ou plus.

36. Complexe selon la revendication 35, dans lequel un lipide bipolaire a des motifs de queue formés par un poly(oxyde d'alkylène) ou un de ses dérivés et l'autre ou les autres a ou ont des motifs de queue formés par un polyol synthétique ou d'origine naturelle.

37. Complexe selon la revendication 36, dans lequel le poly(oxyde d'alkylène) est le poly(oxyde d'éthylène).

38. Complexe selon l'une quelconque des revendications 31 à 37 comprenant en outre au moins une molécule de ciblage.

39. Complexe selon la revendication 38, dans lequel le lipide bipolaire est associé à ladite au moins une molécule de ciblage.

40. Composition comprenant un complexe selon l'une quelconque des revendications 31 à 39 et au moins un autre lipide.

41. Composition selon la revendication 40, dans laquelle ledit au moins un autre lipide est un lipide neutre ou cationique.

42. Composition comprenant un complexe selon l'une quelconque des revendications 31 à 41 et un ou plusieurs véhicules, excipients ou diluants acceptables sur le plan pharmaceutique.

43. Procédé de préparation d'un lipide bipolaire tel que défini dans l'une quelconque des revendications 1 à 28, consistant à coupler :
(A) une tête cationique comprenant deux centres cationiques, ou plus, chaque centre contenant au moins un hétéroatome qui est capable de conserver une charge positive à un pH dans la gamme de 2 à 10 et qui est lié de manière covalente au un ou plusieurs autres centres par un ou plusieurs groupes espaceurs contenant des atomes de carbone ;
(B) un squelette hydrophobe comprenant au moins un chaîne hydrocarbonée ; et
(C) une queue hydrophile comprenant au moins un motif de queue choisi parmi les polyols synthétiques, les polyols d'origine naturelle, les poly(oxyde d'alkylène) et leurs dérivés,
où les matières premières (A), (B) et (C) contiennent un ou plusieurs groupes fonctionnels réactifs appropriés pour faciliter le couplage de sorte que ladite au moins une chaîne hydrocarbonée du squelette hydrophobe est liée de manière covalente à l'un desdits hétéroatomes conservant une charge positive dans la tête cationique et audit au moins un motif de queue dans la queue hydrophile de sorte que la chaîne contient au moins dix atomes liés en une chaîne entre ses points de liaison audit hétéroatome conservant une charge positive et audit au moins un motif de queue.

44. Procédé selon la revendication 43 comprenant l'étape de déprotection d'un dérivé protégé dudit lipide.

45. Procédé de préparation d'un complexe lipidique tel que défini dans l'une quelconque des revendications 31 à 39, comprenant le mélange dudit lipide bipolaire avec une substance bioactive.

46. Utilisation d'un complexe tel que défini dans l'une quelconque des revendications 31 à 39 pour délivrer une substance bioactive à des cellules *in vitro.*

47. Utilisation d'une composition telle que définie dans l'une quelconque des revendications 40 à 42 dans la préparation d'un médicament pour la délivrance d'une substance bioactive.

48. Utilisation selon la revendication 46 ou la revendication 47, dans lequel ladite substance bioactive est un agent thérapeutique, diagnostique ou immunomodulateur.
